# EUROPÄISCHE PATENTSCHRIFT

(11) **EP 0 441 932 B1**
(45) Veröffentlichungstag und Bekanntmachung des Hinweises auf die Patenterteilung: **15.03.1995**
(21) Anmeldenummer: 90912861.3
(22) Anmeldetag: 03.09.1990
(51) Int. Cl.: C07C 43/225, C09K 19/30, C09K 19/12, C09K 19/34, C07C 25/18, C07D 213/64

(54) **FLUORBENZOLDERIVATE UND FLÜSSIGKRISTALLINES MEDIUM**
FLUOROBENZOLE DERIVATIVES AND MESOMORPHOUS MEDIUM
DERIVES FLUOROBENZENIQUES ET MILIEU MESOMORPHE

(30) Priorität: 06.09.1989 DE 3929525; 06.09.1989 DE 3929526; 07.09.1989 DE 3929764; 28.03.1990 DE 4009907
(43) Veröffentlichungstag der Anmeldung: 21.08.1991
(60) Teilanmeldung: 94111142.9
(73) Patentinhaber: MERCK PATENT GmbH, D-64271 Darmstadt (DE)
(72) Erfinder: REIFFENRATH, Volker, D-6101 Rossdorf (DE); KURMEIER, Hans-Adolf, D-6104 Seeheim-Jugenheim (DE); POETSCH, Eike, D-6109 Mühltal 9 (DE); PLACH, Herbert, D-6100 Darmstadt (DE); FINKENZELLER, Ulrich, D-6831 Plankstadt (DE); BARTMANN, Ekkehard, D-6106 Erzhausen (DE); KRAUSE, Joachim, D-6110 Dieburg (DE); SCHEUBLE, Bernhard, D-6104 Seeheim-Jugenheim (DE)
(86) Internationale Anmeldenummer: EP9001471
(87) Internationale Veröffentlichungsnummer: WO9103450

(56) Entgegenhaltungen:
- WO-A-89/02884
- WO-A-90/01056

## Beschreibung

Die Erfindung betrifft neue Fluorbenzolderivate der Formel I, 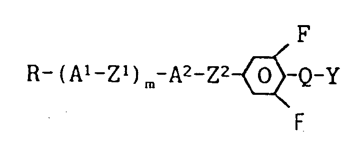
worin
- R: H, einen unsubstituierten, einen einfach durch CN oder CF₃ oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig voneinander durch -O-, -S-, 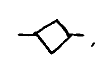 -CO-, -CO-O-, -O-CO- oder -O-CO-O- so ersetzt sein können, daß O-Atome nicht direkt miteinander verknüpft sind,
- A¹ und A²: jeweils unabhängig voneinander einen
(a) trans-1,4-Cyclohexylenrest, worin auch eine oder mehrere nicht benachbarte CH₂-Gruppen durch -O-und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Fluor substituiert sein können,
- Z¹ und Z²: jeweils unabhängig voneinander -CO-O-, -O-CO-, -CH₂O-, -OCH₂-, -CH₂CH₂-, -CH=CH-, -C≡C- oder eine Einfachbindung, einer der Reste Z¹ und Z² auch -(CH₂)₄- oder -CH=CH-CH₂CH₂-,
- m: 0, 1 oder 2,
- Y: F oder Cl, und
- Q: eine Einfachbindung, -CF₂-, -OCF₂- oder -OCHF-
bedeutet, mit der Maßgabe, daß für die Vertragsstaaten AT, BE, CH/LI, DE, FR, GB, IT, LU, NL und SE folgende Verbindungen ausgeschlossen sind:
(a) 4-Propyl-4'-(3,5-difluor-4-difluormethoxyphenyl)-trans, trans-bicyclohexyl
(b) 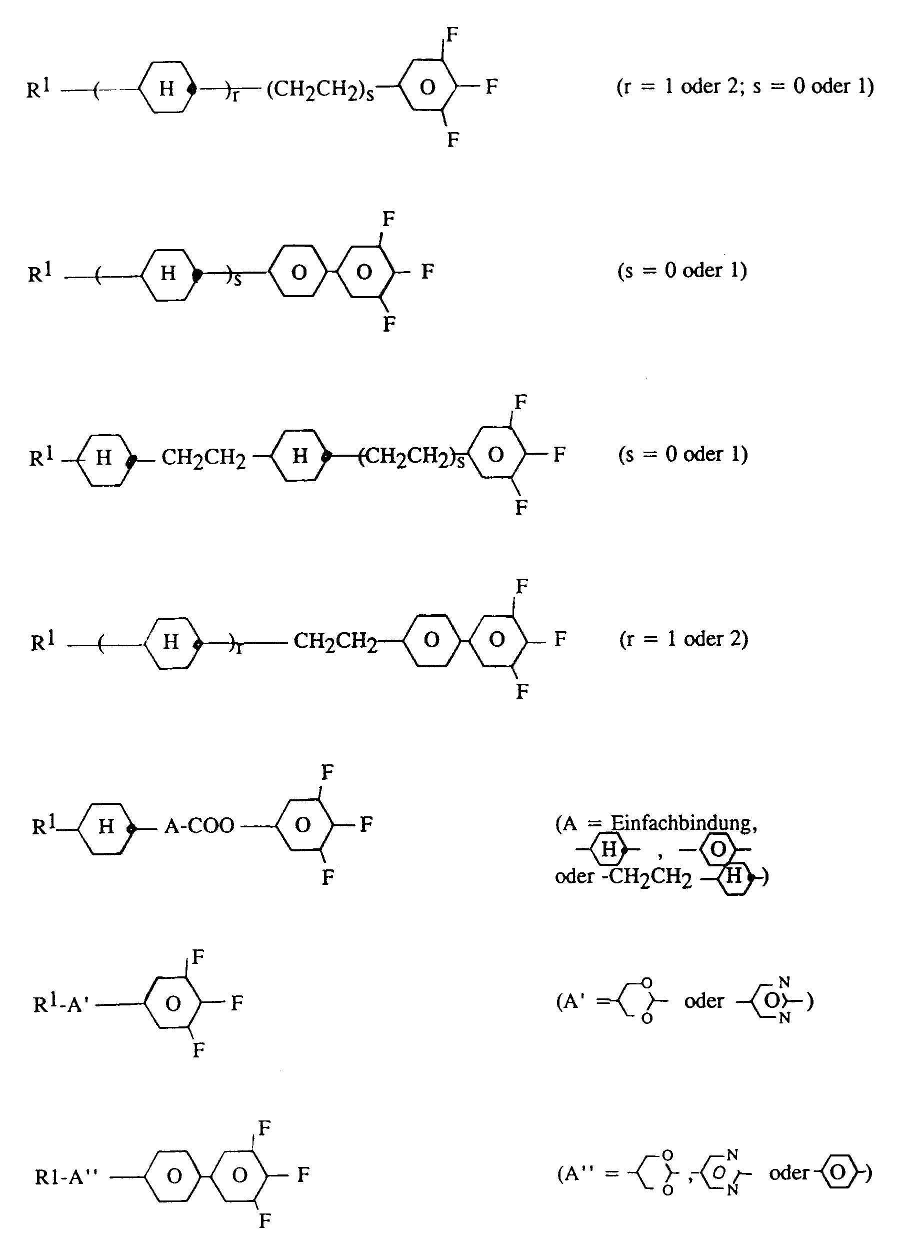
wobei R1 jeweils eine Alkylgruppe mit 1 bis 10 C-Atomen bedeutet, sowie ein flüssigkristallines Medium enthaltend neben einem oder mehreren Fluorbenzolderivaten der Formel I'' 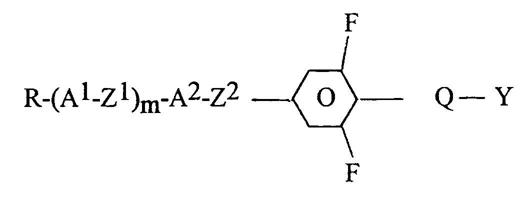
worin
- R: H, einen unsubstituierten, einen einfach durch CN oder CF₃ oder einen mindestens einfach durch Halogen substituierten Alkyl- oder Alkenylrest mit 1 bis 15 C-Atomen wobei in diesen Resten auch eine oder mehrere CH₂-Gruppen jeweils unabhängig voneinander durch -O-, -S-, 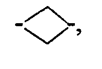 -CO-, -CO-O-, -O-CO- oder -O-CO-O- so ersetzt sein können, daß O-Atome nicht direkt miteinander verknüpft sind,
- A¹ und A²: jeweils unabhängig voneinander einen
(a) trans-1,4-Cyclohexylenrest, worin auch eine oder mehrere nicht benachbarte CH₂-Gruppen durch -O- und/oder -S-ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo (2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Rest (a) und (b) durch CN oder Fluor substituiert sein können,
- Z¹ und Z²: jeweils unabhängig voneinander -CO-O-, -O-CO-, -CH₂O-, -OCH₂-, -CH₂CH₂-, -CH=CH.-, -C≡C-oder eine Einfachbindung, einer der Reste Z1 und Z2 auch -(CH₂)₄- oder -CH=CH-CH₂CH₂-,
- m: 0, 1 oder 2,
- Y: F oder Cl, und
- Q: eine Einfachbindung, -CF₂-, -OCF₂- oder -OCHF-
bedeutet, als weitere Bestandteile 4 bis 30 Komponenten.

Die Erfindung betrifft weiterhin die Verwendung der Verbindungen der Formel I als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine relativ hohe dielektrische Anisotropie.

Es wurde nun gefunden, daß Verbindungen der Formel I als Komponenten flüssigkristalliner Medien vorzüglich geeignet sind, Insbesondere verfügen sie über vergleichsweise niedrige Viskositäten. Mit ihrer Hilfe lassen sich stabile flüssig- kristalline Medien mit breitem Mesophasenbereich und vorteilhaften Werten für die optische und dielektrische Anisotropie erhalten. Diese Medien weisen ferner ein sehr gutes Tieftemperaturverhalten auf.

Flüssigkristalle der Formel 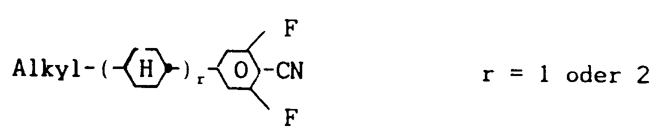
sind bereits aus DE 3209178 bekannt. Aus der JP 62-103057 sind Verbindungen der Formeln 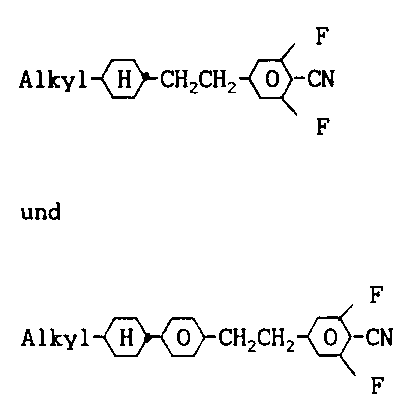
bekannt. In JP 63-216858 schließlich werden Verbindungen der Formel 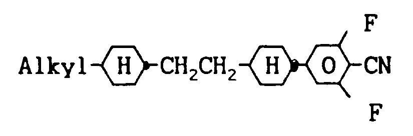
beschrieben. Aus den DE-OS 30 42 391 und DE-OS 31 39 130 sind Verbindungen der folgenden Formeln bekannt: 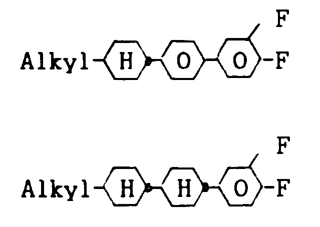
Verschiedene Verbindungen mit flüssigkristallinen Eigenschaften, in denen terminal eine CF₃-Gruppe gebunden ist, sind bereits bekannt (USP 4,330,426; USP 4,684,476; J.C. Liang and S. Kumar, Mol. Cryst. Liq. Cryst. 1987; Vol. 142, pp. 77-84). Diese Verbindungen haben jedoch oft einen stark smektogenen Charakter und sind für viele praktische Anwendungen weniger geeignet.

Im Hinblick auf die verschiedensten Einsatzbereiche derartiger Verbindungen mit hohem Δε war es jedoch wünschenswert, weitere Verbindungen zur Verfügung zu haben, die auf die jeweiligen Anwendungen genau maßgeschneiderte Eigenschaften aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I bzw. flüssigkristalline Medien enthaltend eine oder mehrere Verbindungen der Formel I'' und 4 bis 30 weitere Bestandteile sowie

Flüssigkristallanzeigeelemente, insbesondere elektroopische Anzeigeelemente, die derartige Medien enthalten.

Der Einfachheit halber bedeuten im folgenden A³ einen Rest der Formel 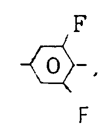
Cyc einen 1,4-Cyclohexylenrest, Che einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bi einen Bicyclo(2,2,2)-octylenrest, wobei Cyc und/oder Phe unsubstituiert oder ein- oder zweifach durch F oder CN substituiert sein können. Y ist vorzugsweise F.

A₁ und A₂ sind vorzugsweise ausgewählt aus der Gruppe Cyc, Che, Phe, Pyr, Pyd und Dio, wobei Vorzugsweise nur einer der im Molekül vorhandenen Reste A₁ und A₂ Che, Phe, Pyr, Pyd oder Dio ist.

Die Verbindungen der Formel I umfassen dementsprechend Verbindungen mit zwei Ringen der Teilformeln Ia und Ib:

R-A²-A³-Q-Y Ia

R-A²-Z²-A³-Q-Y Ib

Verbindungen mit drei Ringen der Teilformeln Ic bis If:

R-A¹-A²-A³-Q-Y Ic

R-A¹-Z¹-A²-Z²-A³-Q-Y Id

R-A¹-Z¹-A²-A³-Q-Y Ie

R-A¹-A²-Z²-A³-Q-Y If

sowie Verbindungen mit vier Ringen der Teilformeln Ig bis Im:

R-A¹-A¹-A²-A³-Q-Y Ig

R-A¹-Z¹-A¹-A²-A³-Q-Y Ih

R-A¹-A¹-Z¹-A²-A³-Q-Y Ii

R-A¹-A¹-A²-Z¹-A³-Q-Y Ij

R-A¹-Z¹-A¹-Z¹-A²-A³-Q-Y Ik

R-A¹-A¹-Z¹-A²-Z²-A³-Q-Y Il

R-A¹-Z¹-A¹-Z¹-A²-Z²-A³-Q-Y Im

Darunter sind besonders diejenigen der Teilformeln Ia, Ib, Ic, Id, Ie, If, Ii und Il bevorzugt.

Die bevorzugten Verbindungen der Teilformel Ia umfassen diejenigen der Teilformeln Iaa bis Iah:

R-Phe-A³-Q-Y Iaa

R-Phe-A³-Q-Y Iab

R-Dio-A³-Q-Y Iac

R-Pyr-A³-Q-Y Iad

R-Pyd-A³-Q-Y Iae

R-Cyc-A³-Q-Y Iaf

R-Cyc-A³-Q-Y Iag

R-Che-A³-Q-Y Iah

Darunter sind diejenigen der Formeln Iaa, Iab, Iac, Iad, Iaf und Iag besonders bevorzugt.

Die bevorzugten Verbindungen der Teilformel Ib umfassen diejenigen der Teilformeln Iba und Ibb:

R-Cyc-CH₂CH₂-A³-Q-Y Iba

R-Cyc-COO-A³-Q-Y Ibb

Die bevorzugten Verbindungen der Teilformel Ic umfassen diejenigen der Teilformeln Ica bis Ico:

R-Phe-Phe-A³-Q-Y Ica

R-Phe-Phe-A³-Q-Y Icb

R-Phe-Dio-A³-Q-Y Icc

R-Cyc-Cyc-A³-Q-Y Icd

R-Phe-Cyc-A³-Q-Y Ice

R-Cyc-Cyc-A³-Q-Y Icf

R-Pyd-Phe-A³-Q-Y Icg

R-Pyr-Phe-A³-Q-Y Ich

R-Phe-Pyr-A³-Q-Y Ici

R-Cyc-Pyr-A³-Q-Y Icj

R-Cyc-Phe-A³-Q-Y Ick

R-Cyc-Phe-A³-Q-Y Icl

R-Dio-Phe-A³-Q-Y Icm

R-Che-Phe-A³-Q-Y Icn

R-Phe-Che-A³-Q-Y Ico

Darunter sind diejenigen der Formeln Ica, Icc, Icd, Ice, Ici und Icj besonders bevorzugt.

Die bevorzugten Verbindungen der Teilformel Id umfassen diejenigen der Teilformeln Ida bis Idm:

R-Phe-Z¹-Phe-Z¹-A³-Q-Y Ida

R-Phe-Z¹-Phe-Z¹-A³-Q-Y Idb

R-Phe-Z¹-Dio-Z¹-A³-Q-Y Idc

R-Cyc-Z¹-Cyc-Z¹-A³-Q-Y Idd

R-Cyc-Z¹-Cyc-Z¹-A³-Q-Y Ide

R-Pyd-Z¹-Phe-Z¹-A³-Q-Y Idf

R-Phe-Z¹-Pyd-Z¹-A³-Q-Y Idg

R-Pyr-Z¹-Phe-Z¹-A³-Q-Y Idh

R-Phe-Z¹-Pyr-Z¹-A³-Q-Y Idi

R-Phe-Z¹-Cyc-Z¹-A³-Q-Y Idj

R-Cyc-Z¹-Phe-Z¹-A³-Q-Y Idk

R-Cyc-Z¹-Phe-Z¹-A³-Q-Y Idl

R-Dio-Z¹-Phe-Z¹-A³-Q-Y Idm

Die bevorzugten Verbindungen der Teilformel Ie umfassen diejenigen der Teilformeln Iea bis Iel:

R-Pyr-Z¹-Phe-A³-Q-Y Iea

R-Dio-Z¹-Phe-A³-Q-Y Ieb

R-Phe-Z¹-Phe-A³-Q-Y Iec

R-Cyc-Z¹-Phe-A³-Q-Y Ied

R-Cyc-Z¹-Phe-A³-Q-Y Iee

R-Phe-Z¹-Cyc-A³-Q-Y Ief

R-Cyc-Z¹-Cyc-A³-Q-Y Ieg

R-Cyc-Z¹-Cyc-A³-Q-Y Ieh

R-Phe-Z¹-Dio-A³-Q-Y Iei

R-Pyd-Z¹-Phe-A³-Q-Y Iej

R-Phe-Z¹-Pyr-A³-Q-Y Iek

R-Cyc-Z¹-Pyr-A³-Q-Y Iel

Die bevorzugten Verbindungen der Teilformel If umfassen diejenigen der Teilformeln Ifa bis Ifr:

R-Pyr-Phe-Z¹-A³-Q-Y Ifa

R-Pyr-Phe-OCH₂-A³-Q-Y Ifb

R-Phe-Phe-Z¹-A³-Q-Y Ifc

R-Phe-Phe-OOC-A³-Q-Y Ifd

R-Phe-Phe-Z¹-A³-Q-Y Ife

R-Cyc-Cyc-Z¹-A³-Q-Y Iff

R-Cyc-Cyc-Z¹-A³-Q-Y Ifg

R-Cyc-Cyc-CH₂CH₂-A³-Q-Y Ifh

R-Pyd-Phe-Z¹-A³-Q-Y Ifi

R-Dio-Phe-Z¹-A³-Q-Y Ifj

R-Phe-Cyc-Z¹-A³-Q-Y Ifk

R-Phe-Cyc-Z¹-A³-Q-Y Ifl

R-Phe-Pyd-Z¹-A³-Q-Y Ifm

R-Che-Phe-Z¹-A³-Q-Y Ifn

R-Phe-Che-Z¹-A³-Q-Y Ifo

R-Cyc-Phe-Z¹-A³-Q-Y Ifp

R-Cyc-Phe-OOC-A³-Q-Y Ifq

R-Cyc-Phe-Z¹-A³-Q-Y Ifr

In den Verbindungen der vor- und nachstehenden Formeln bedeutet Y vorzugsweise F.

R bedeutet vorzugsweise Alkyl, ferner Alkoxy. A¹ und/oder A² bedeuten bevorzugt Phe, Cyc, Che, Pyr oder Dio. Bevorzugt enthalten die Verbindungen der Formel I nicht mehr als einen der Reste Bi, Pyd, Pyr, Dio oder Dit.

Bevorzugt sind auch Verbindungen der Formel I sowie aller Teilformeln, in denen A¹ und/oder A² ein- oder zweifach durch F oder einfach durch CN substituiertes 1,4-Phenylen bedeutet. Insbesondere sind dies 2-Fluor-1,4-phenylen, 3-Fluor-1,4-phenylen und 3,5-Difluor-1,4-phenylen sowie 2-Cyan-1,4-phenylen und 3-Cyan-1,4-phenylen. In einer besonders bevorzugten Ausführungsform ist A² 3,5-Difluor-1,4-phenylen und m 1 oder 2.

Z¹ und Z² bedeuten bevorzugt eine Einfachbindung, -CO-O-, -O-CO- und -CH₂CH₂-, in zweiter Linie bevorzugt -CH₂O- und -OCH₂-.

Falls einer der Reste Z¹ und Z² -(CH₂)₄- oder -CH=CH-CH₂CH₂-bedeutet, so ist der andere Rest Z¹ oder Z² (falls vorhanden) vorzugsweise die Einfachbindung.

Bevorzugte Verbindungen dieses Types entsprechen der Teilformel I' 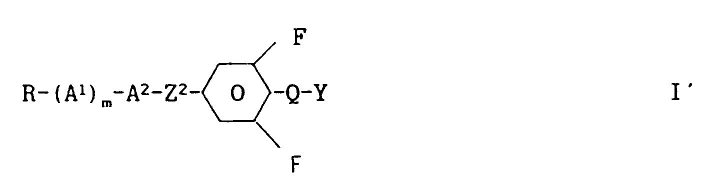
worin Z² -(CH₂)₄- oder -CH=CH-CH₂CH₂- bedeutet und R, A¹, A², m, Q und Y die bei Formel I angegebene Bedeutung haben, Auch die bevorzugten Bedeutungen für R, A¹, A², m, Q und Y entsprechen denen für die Verbindungen der Formel I.

m ist vorzugsweise 1 oder 0, insbesondere bevorzugt 0.

Falls R einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine CH₂-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkylrest bedeutet, in dem eine CH₂-Gruppe durch unsubstituiertes oder substituiertes -CH=CH- und eine benachbarte CH₂-Gruppe durch CO oder CO-O oder O-CO-ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxy-propyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R einen einfach durch CN oder CF₃ substituierten Alkyl- oder Alkenylrest bedeutet, so ist dieser Rest vorzugsweise geradkettig und die Substitution durch CN oder CF₃ in ω-Position.

Falls R einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sei, vorzugsweise jedoch in ω-Position.

Verbindungen der Formel I, die über für Polymerisationsreaktionen geeignete Flügelgruppen R verfügen, eignen sich zur Darstellung flüssigkristalliner Polymerer.

Verbindungen der Formeln I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxy-carbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Verbindungen der Formel I, die über für Polykondensationen geeignete Flügelgruppen R verfügen, eignen sich zur Darstellung flüssigkristalliner Polykondensate.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Einige ganz besonders bevorzugte kleinere Gruppen von Verbindungen sind diejenigen der Teilformeln I1 bis I11: 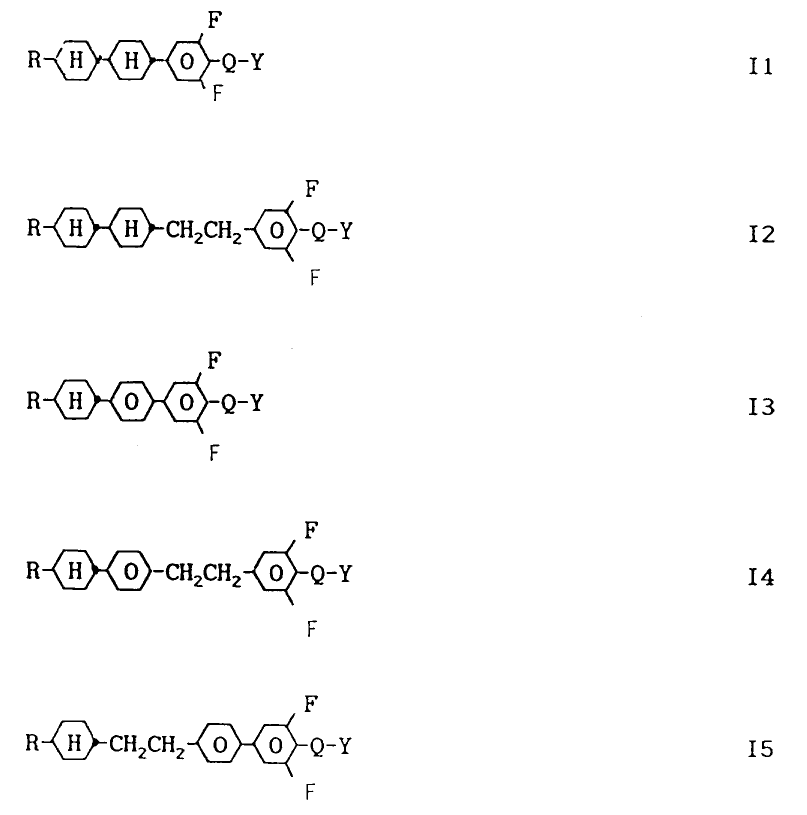 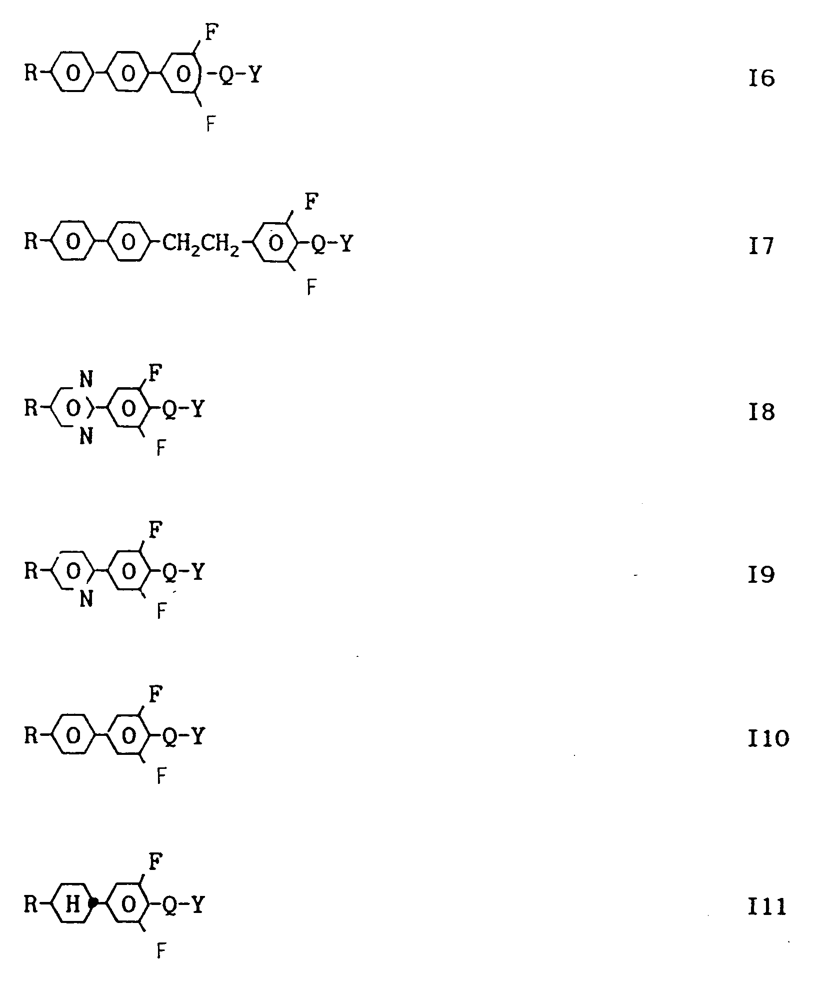
Die 1,4-Cyclohexenylen-Gruppe hat vorzugsweise folgende Strukturen: 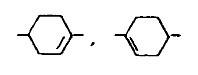
Die Verbindungen der Formel I bzw. I'' werden nach an sich bekannten Methoden dargestellt, wie sie in der Literatur (z.B. in den Standardwerken wie Houben-Weyl, Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind.

Dabei kann man auch von an sich bekannten, hier nicht näher erwähnten Varianten Gebrauch machen.

Die Verbindungen der Formel I bzw. I'' können z. B. hergestellt, indem man eine Verbindung der Formel II, 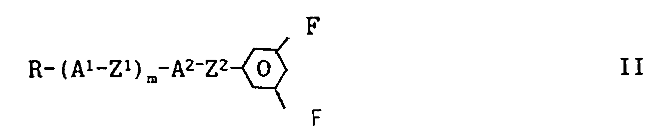
worin R, A¹, A², Z¹, Z² und m die angegebene Bedeutung haben, gemäß folgendem Reaktionsschema metalliert und anschließend mit einem geeigneten Elektrophil umsetzt: 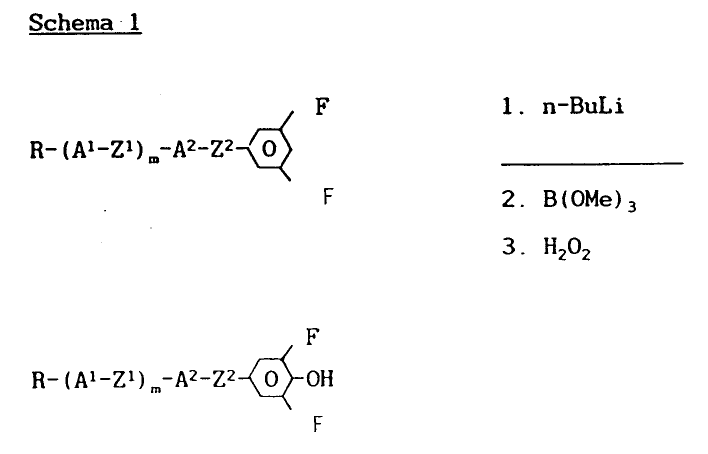
Aus dem erhaltenen Phenol sind die Zielprodukte mit Q = OCF₂ oder OCHF nach bekannten Methoden, z. B. durch Umsetzung mit Chlordifluormethan bzw. Tetrachlorkohlenstoff/HF erhältlich.

Weitere Synthesemethoden sind für den Fachmann augenscheinlich. Beispielsweise können in 5-Position entsprechend substituierte 1,3-Difluorbenzol-Verbindungen gemäß obigem Schema in die 2-OCF₂Y-1,3-difluor-Verbindungen überführt werden und der Rest R-(A¹-Z¹)ₘA²-Z² anschließend durch in der Flüssigkristallchemie gebräuchliche Reaktionen (z.B. Veresterung, Veretherung oder Kopplungen z.B. gemäß der Artikel E. Poetsch, Kontakte (Darmstadt) 1988 (2), S. 15) eingeführt werden. 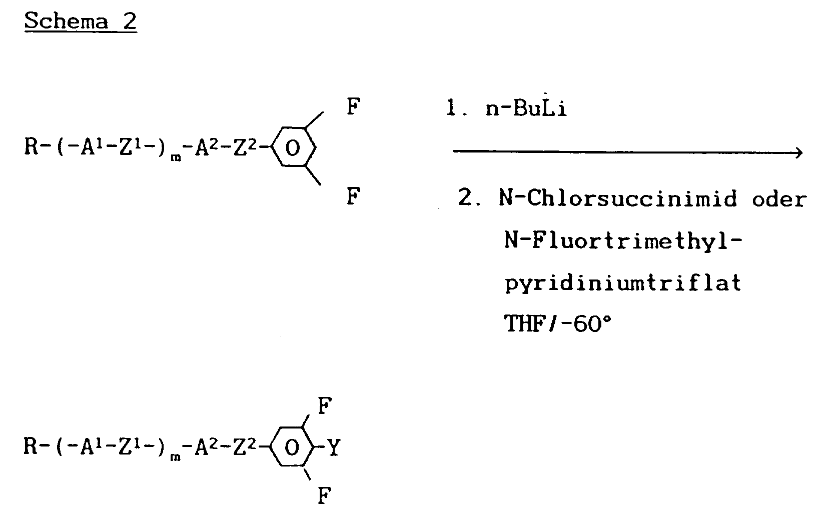
Weitere Synthesemethoden sind für den Fachmann augenscheinlich. Beispielsweise können in 5-Position entsprechend substituierte 1,3-Difluorbenzol-Verbindungen gemäß obigem Schema in die 2-Y-1,3-difluor-Verbindungen überführt werden und der Rest R-(A¹-Z¹)ₘ-A²-Z² anschließend durch in der Flüssigkristallchemie gebräuchliche Reaktionen (z.B. Veresterung, Veretherung oder Kopplungen z.B. gemäß der Artikel E. Poetsch, Kontakte (Darmstadt) 1988 (2), S. 15) angeführt werden.

Die erfindungsgemäßen Verbindungen der Formel I, worin Q-Y CF₃ bedeutet, lassen sich durch Metallierung der unsubstituierten 3,5-Difluorphenylverbindungen mit n-BuLi, anschließender Reaktion mit Jod und Umsetzung der Jodverbindung mit Trifluoressigsäure-Natriumsalz gemäß folgenden Schema herstellen: 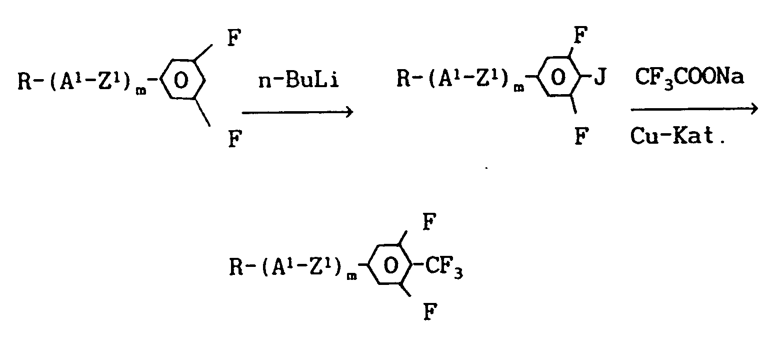
Die Verbindungen der Formel II können beispielsweise nach folgenden Syntheseschemata hergestellt werden: 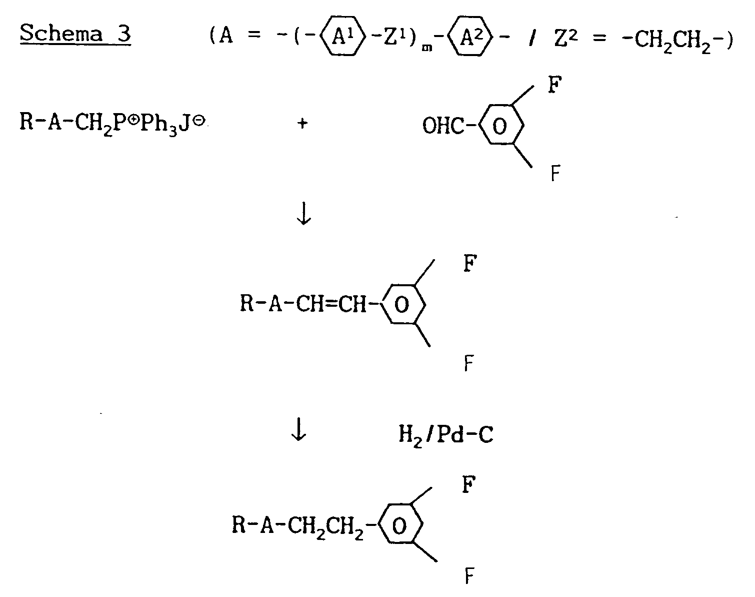 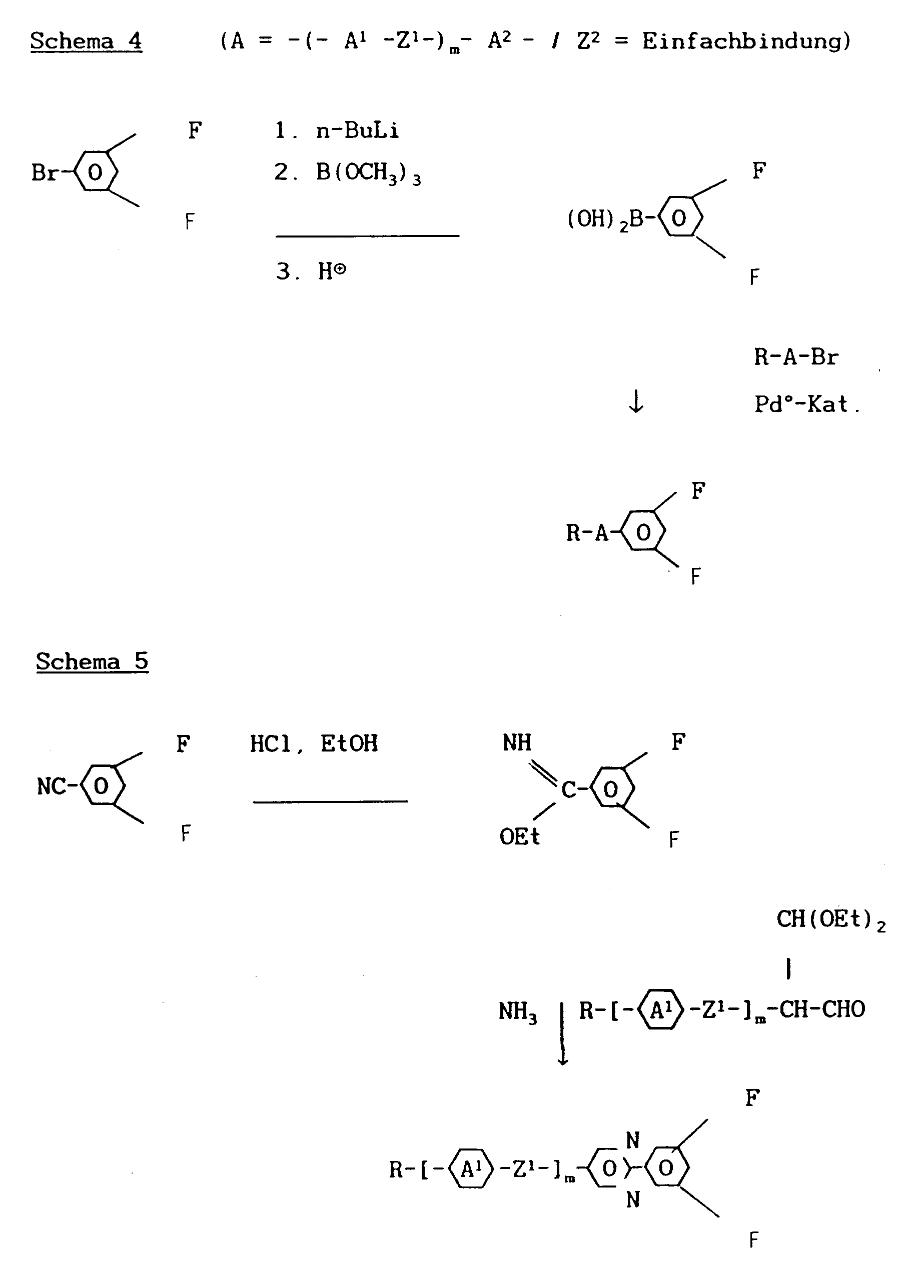 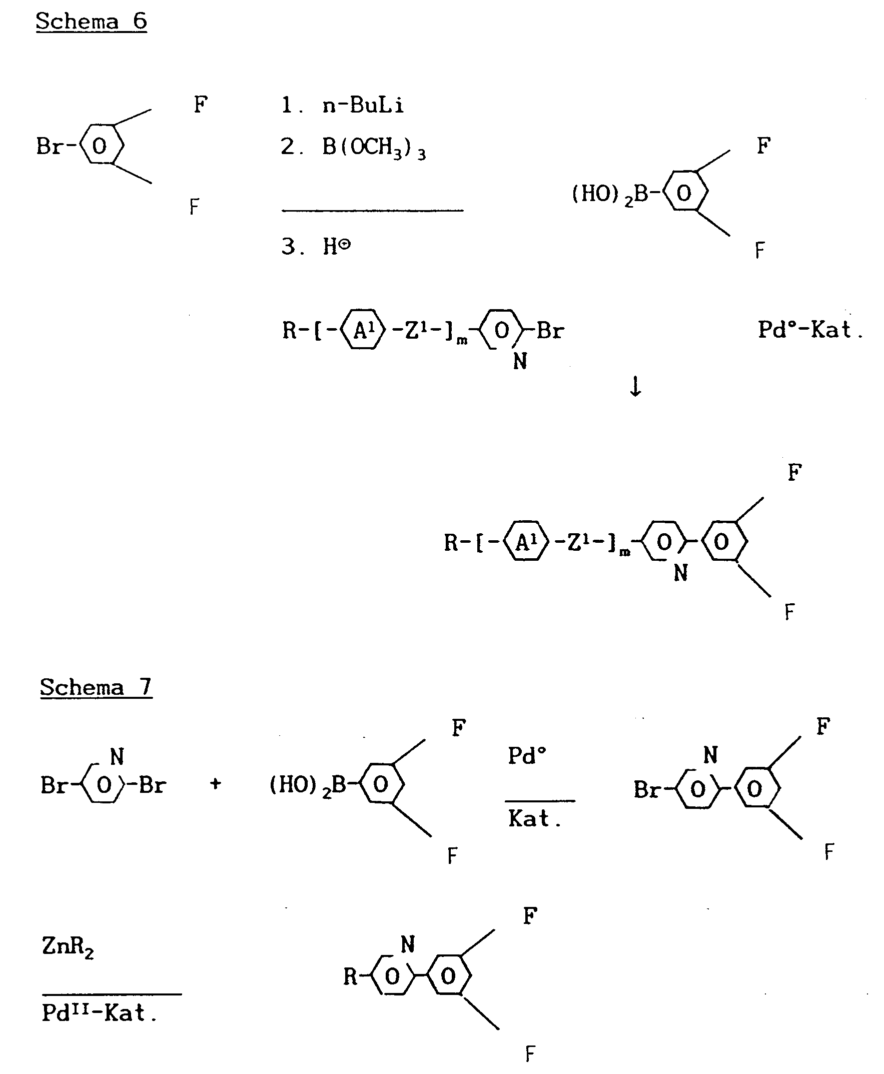
Die Ausgangsmaterialien sind entweder bekannt oder können in Analogie zu bekannten Verbindungen hergestellt werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

Die Synthese einiger besonders bevorzugter Verbindungen ist im folgenden näher angegeben: 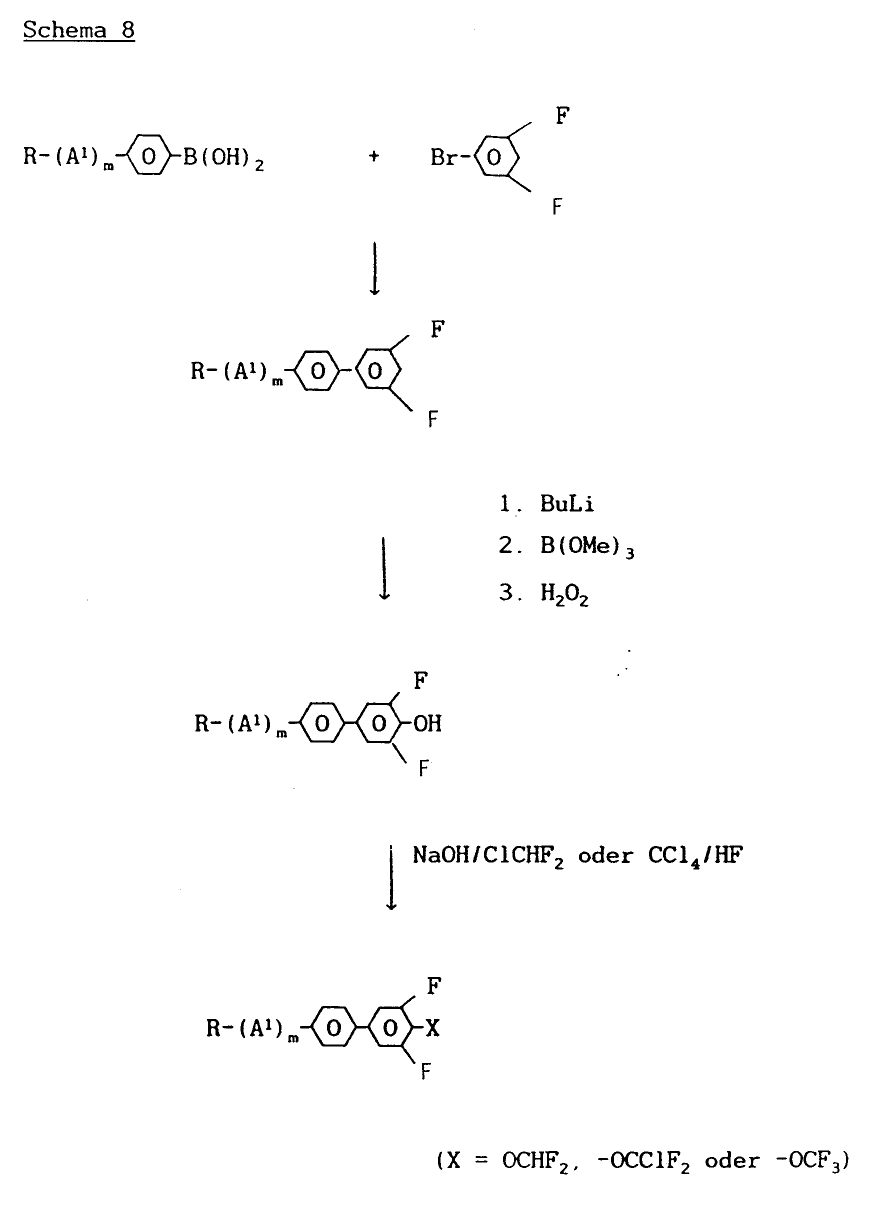 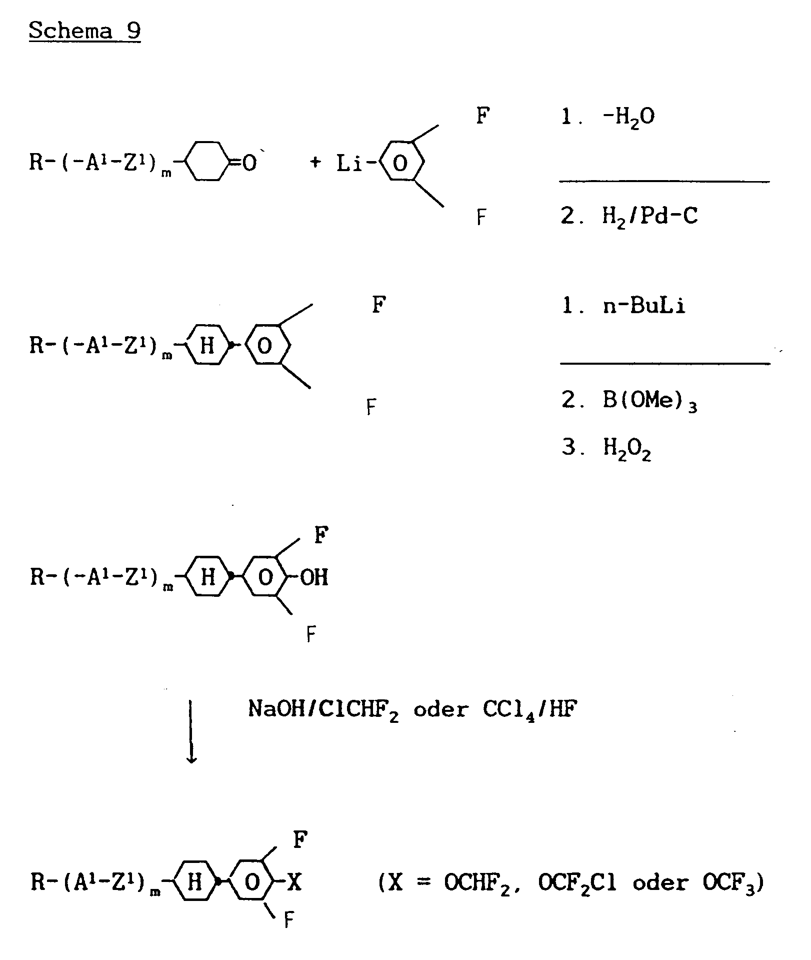
In Schemata 8 und 9 ist vorzugsweise m 0 oder 1 und 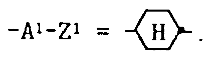 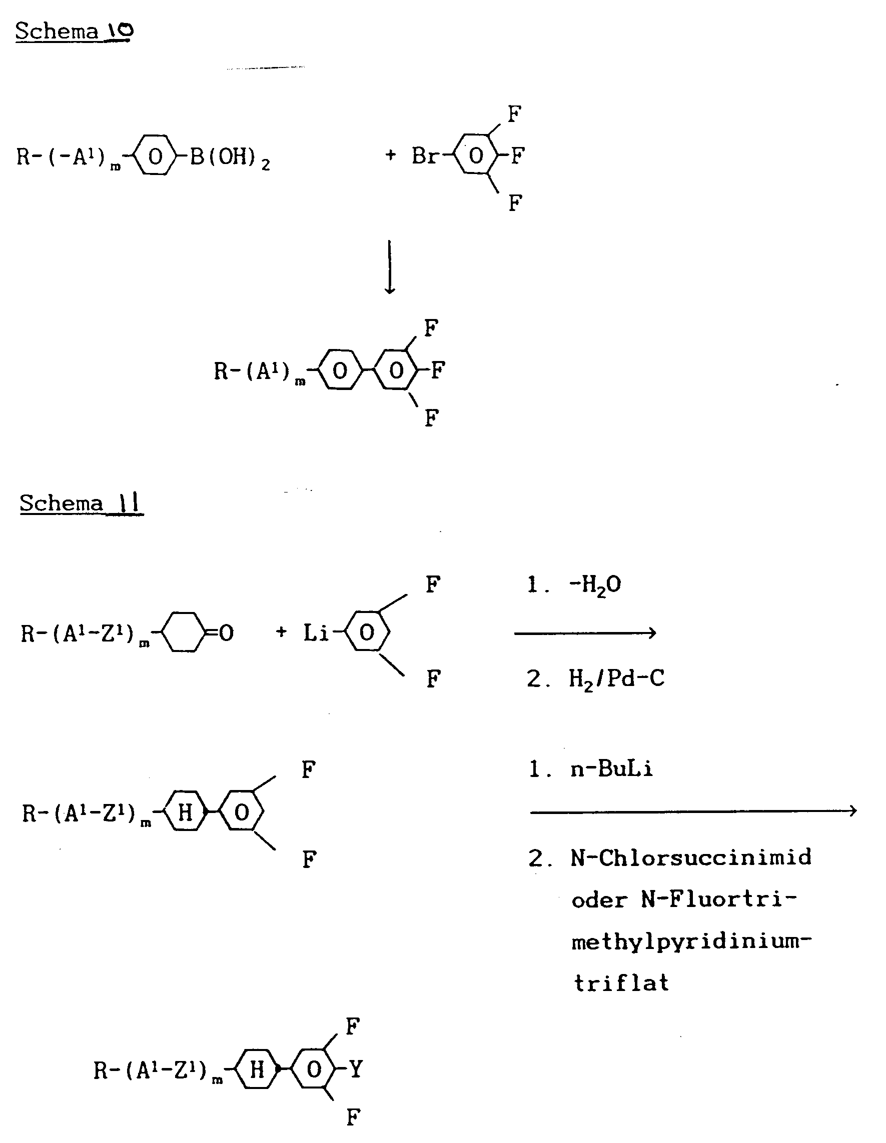 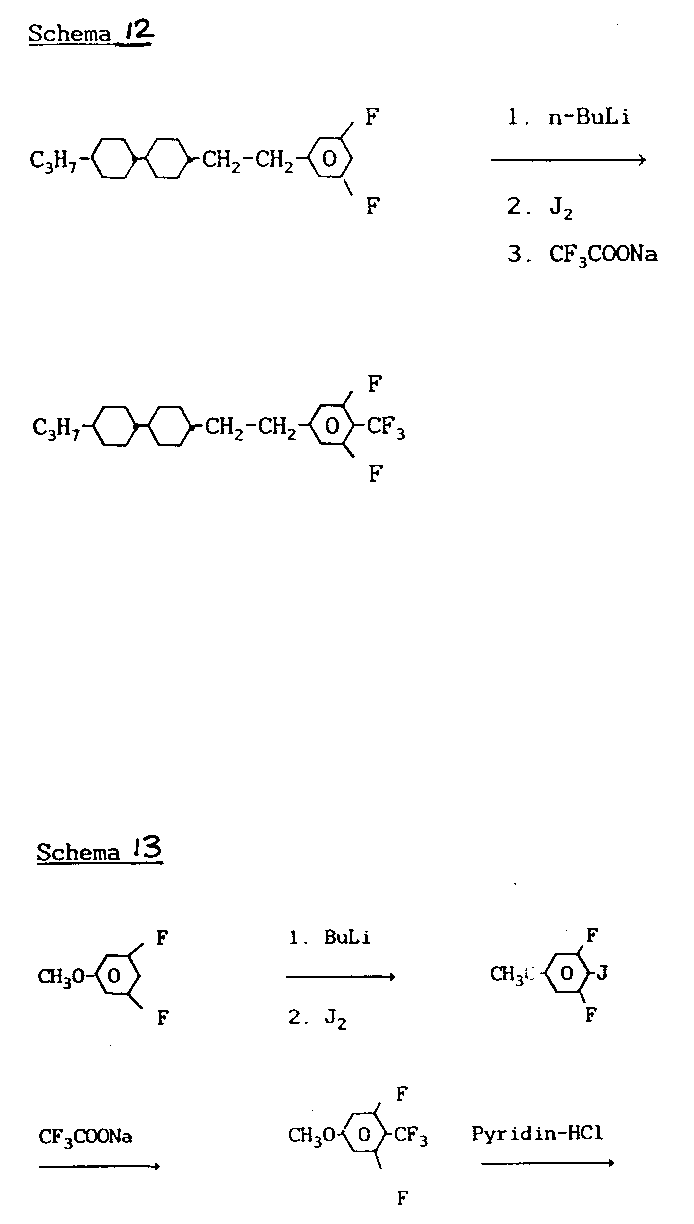 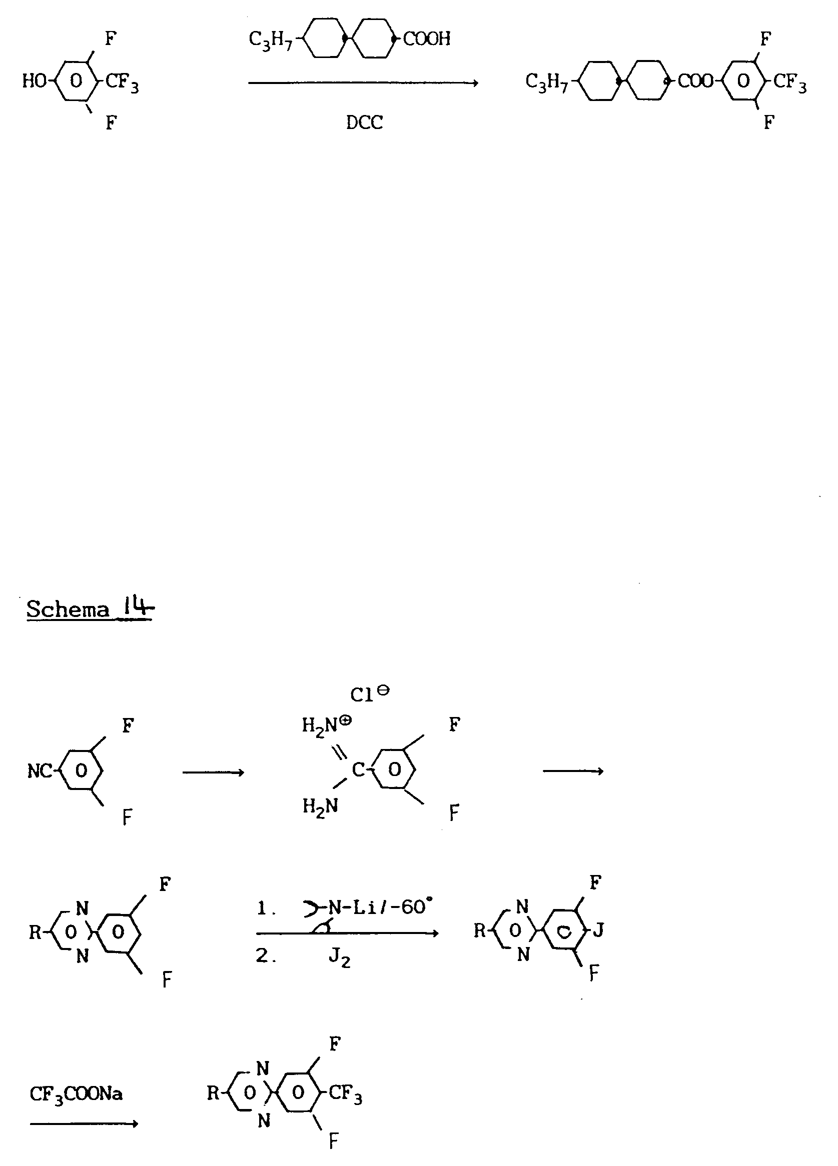
Ester der Formel I können auch durch Verestorung entsprechender Carbonsäuren (oder ihrer raktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

In einem weiteren Verfahren zur Herstellung der Verbindungen der Formel I setzt man ein Arylhalogenid mit einem Olefin um in Gegenwart eines tertiären Amins und eines Palladiumkatalysators (vgl. R.F. Heck, Acc. Chem. Res. 12 (1979) 146). Geeignete Arylhalogenide sind beispielsweise Chloride, Bromide und Iodide, insbesondere Bromide und Iodide. Die für das Gelingen der Kupplungsreaktion erforderlichen tertiären Amine, wie z.B. Triethylamin, eignen sich auch als Lösungsmittel. Als Palladiumkatalysatoren sind beispielsweise dessen Salze, insbesondere (Pd(II)-acetat, mit organischen Phosphor(III)-Verbindungen wie z.B. Triarylphosphanen geeignet. Man kann dabei in Gegenwart oder Abwesenheit eines inerten Lösungsmittels bei Temperaturen zwischen etwa 0° und 150°, vorzugsweise zwischen 20° und 100°, arbeiten; als Lösungsmittel kommen z.B. Nitrile wie Acetonitrile oder Kohlenwasserstoffe wie Benzol oder Toluol in Betracht. Die als Ausgangsstoffe eingesetzten Arylhalogenide und Olefine sind vielfach im Handel erhältlich oder können nach literaturbekannten Verfahren hergestellt werden, beispielsweise durch Halogenierung entsprechender Stammverbindungen bzw. durch Eliminierungsreaktionen an entsprechenden Alkoholen oder Halogeniden.

Auf diese Weise sind beispielsweise Stilbenderivate herstellbar. Die Stilbene können weiterhin hergestellt werden durch Umsetzung eines 4-sübstituierten Benzaldehyds mit einem entsprechenden Phoshorylid nach Wittig. Man kann aber auch Tolane der Formel I herstellen, indem man anstelle des Olefins monosubstituiertes Acetylen einsetzt (Synthesis 627 (1980) oder Tetrahedron Lett. 27, 1171 (1986)).

Weiterhin können zur Kopplung von Aromaten Arylhalogenide mit Arylzinnverbindungen umgesetzt werden. Bevorzugt werden diese Reaktionen unter Zusatz eines Katalysators wie z.B. eines Palladium(O)komplexes in inerten Lösungsmitteln wie Kohlenwasserstoffen bei hohen Temperaturen, z.B. in siedendem Xylol, unter Schutzgas durchgeführt.

Kopplungen von Alkinyl-Verbindungen mit Arylhalogeniden können analog dem von A.O. King, E. Negishi, F.J. Villani und A. Silveira in J. Org. Chem. 43, 358 (1978) beschriebenen Verfahren durchgeführt werden.

Tolane der Formel I können auch über die Fritsch-Buttenberg-Wiechell-Umlagerung (Ann. 279, 319, 1984) hergestellt werden, bei der 1,1-Diaryl-2-halogenethylene umgelagert werden zu Diarylacetylenen in Gegenwart starker Basen.

Tolane der Formel I können auch hergestellt werden, indem man die entsprechenden Stilbene bromiert und anschließend einer Dehydrohalogenierung unterwirft. Dabei kann man an sich bekannte, hier nicht näher erwähnte Varianten dieser Umsetzung anwenden.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie z. B. Aceton, 1,2-Dimethoxyethan, DMF oder Dimethylsulfoxid oder auch mit einem Überschüß an wäßriger oder wäßrig-alkoholischer NaOH oder KOH bei Temperaturen zwischen etwa 20° und 100°.

Die Ausgangsmaterialien sind entweder bekannt oder können in Analogie zu bekannten Verbindungen hergestellt werden.

Die Verbindungen der Formel I' mit Z² = -(CH₂)₄- können nach folgendem Schema hergestellt werden: 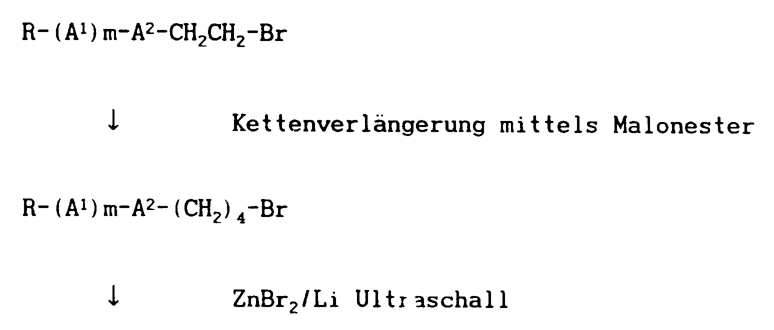 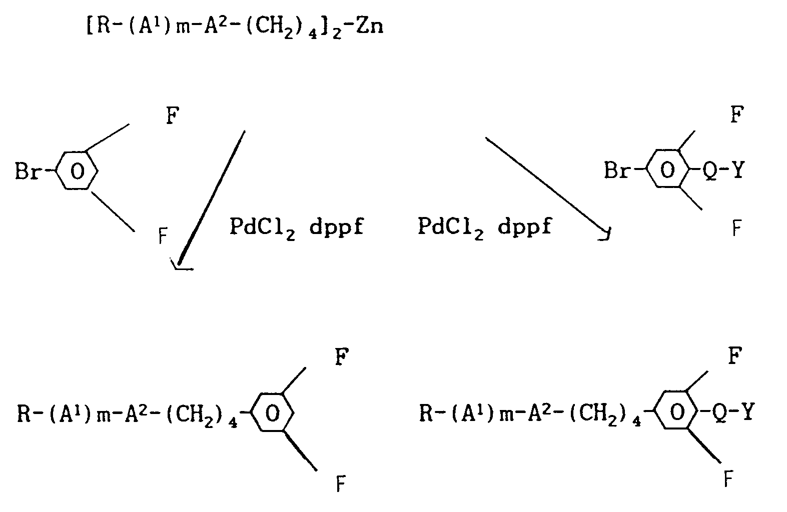
Bei der Pd(II)-katalysierten Kopplungsreaktion wird entweder direkt das Zielprodukt I' gebildet oder ein Vorprodukt, in das völlig analog zu den vorstehenden Methoden für Verbindungen oder Formel I der Rest -Q-Y eingeführt wird.

Die Verbindungen der Formel I' mit Z² = -CH=CH-CH₂CH₂- können noch Wittig gemäß folgendem Schema hergestellt werden:

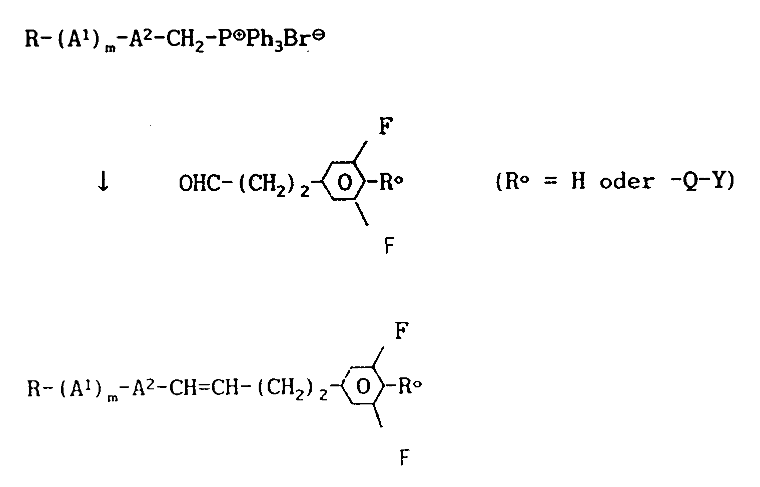

Die bevorzugten trans-Isomeren können nach können nach den literaturbekannten Isomerisierungsmethoden hergestellt werden. Die ggf. erhaltenen Vorprodukte mit R° = H werden völlig analog zu den Vorprodukten der Verbindungen der Formel I durch Einführen des Restes -Q-Y in die Verbindungen der Formel I' übergeführt.

Die Aldehyde können durch Heck-Reaktion von entsprechend substituierten 1-Brom-3-fluorbenzolderivaten mit Allylalkohol erhalten werden.

Die erfindungsgemäßen flüssigkristallinen Medien enthalten vorzugsweise neben einer oder mehreren erfindungsgemäßen Verbindungen bzw. neben einer oder mehreren Verbindungen der Formel I'' als weitere Bestandteile 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäure-phenyl- oder cyclohexyl-ester, Phenyl- oder Cyclohexyl-ester der Cyclohexylbenzoesäure, Phenyl- oder Cyclohexyl-ester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Bis-cyclohexylbenzole 4,4'-Bis-cyclohexylbiphenyle, Phenyl- oder Cyclohexylpyrimidine. Phenyl- oder oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexy-2-(4-phenyl-cyclohexyl)-ethane, 1-Cyclohexyl-2-biphenylylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbidnungen lassen sich durch die Formeln 1, 2, 3, 4 und 5 charakterisieren:

R'-L-E-R'' 1

R'-L-COO-E-R'' 2

R'-L-OOC-E-R'' 3

R'-L-CH₂CH₂-E-R'' 4

R'-L-C≡C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc-sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2-5-diyl oder Pyridin-2,5-diyl, Bio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in einer kleineren Untergruppe der verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R'' voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R'' -F, -Cl, -NCS oder -(O)ᵢ CH₃₋₍ₖ₊₁₎ FₖCl₁, wobei i 0 oder 1 und k+1 1, 2 oder 3 sind; die Verbindungen, in denen R'' diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R'' die Bedeutung -F, -Cl, -NCS, -CF₃, OCHF₂ oder -OCF₃ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R'' -CN; diese Untergruppe wird im folgenden als Gruppe C bezeichnet und die Verbindungen dieser Untergruppe werden entsprechend mit Teilformeln 1c, 2c, 3c, 4c und 5 c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben Verbindungen der Formel I bzw. I'' vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise
- Gruppe A:: 0 bis 90 %, vorzugsweise 20 bis 90 %, insbesondere 30 bis 90 %
- Gruppe B:: 0 bis 80 %, vorzugsweise 10 bis 80 %, insbesondere 10 bis 65 %
- Gruppe C:: 0 bis 80 %, vorzugsweise 5 bis 80 %, insbesondere 5 bis 50 %
wobei die Summe der Massenanteile der in den jeweiligen erfindungsgemäßen Medien enthaltenen Verbindungen aus den Gruppen A und/oder B und/oder C vorzugsweise 5 %-90 % und insbesondere 10 % bis 90 % beträgt.

Die erfindungsgemäßen Medien enthalten vorzugsweise 1 bis 40 %, insbesondere 5 bis 30 % an Verbindungen der Formel I bzw. I''. Weiterhin bevorzugt sind Medien, enthaltend mehr als 40 %, insbesondere 45 bis 90 % an Verbindungen der Formel I bzw. I''. Die Medien enthalten vorzugsweise drei, vier oder fünf Verbindungen der Formel I bzw. I''.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroiti sche Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. Vor- und nachstehend bedeuten Potenzangaben Gewichtsprozent. Alle Temperaturen sind in Grad Celsius angegeben. Fp. bedeutet Schmelzpunkt Kp = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Viskosität (mm²/sec) wurde bei 20 °C bestimmt.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Rest CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| nT | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nOmFF | CₙH₂ₙ₊₁ | OCₘH₂ₘ₊₁ | F | F |
| nmF | CₙH₂ₙ₊₁ | CₘH₂ₘ₊₁ | F | H |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nNF | CₙH₂ₙ₊₁ | CN | F | H |
| nAm | CₙH₂ₙ₊₁ | COOCₘH₂ₘ₊₁ | H | H |

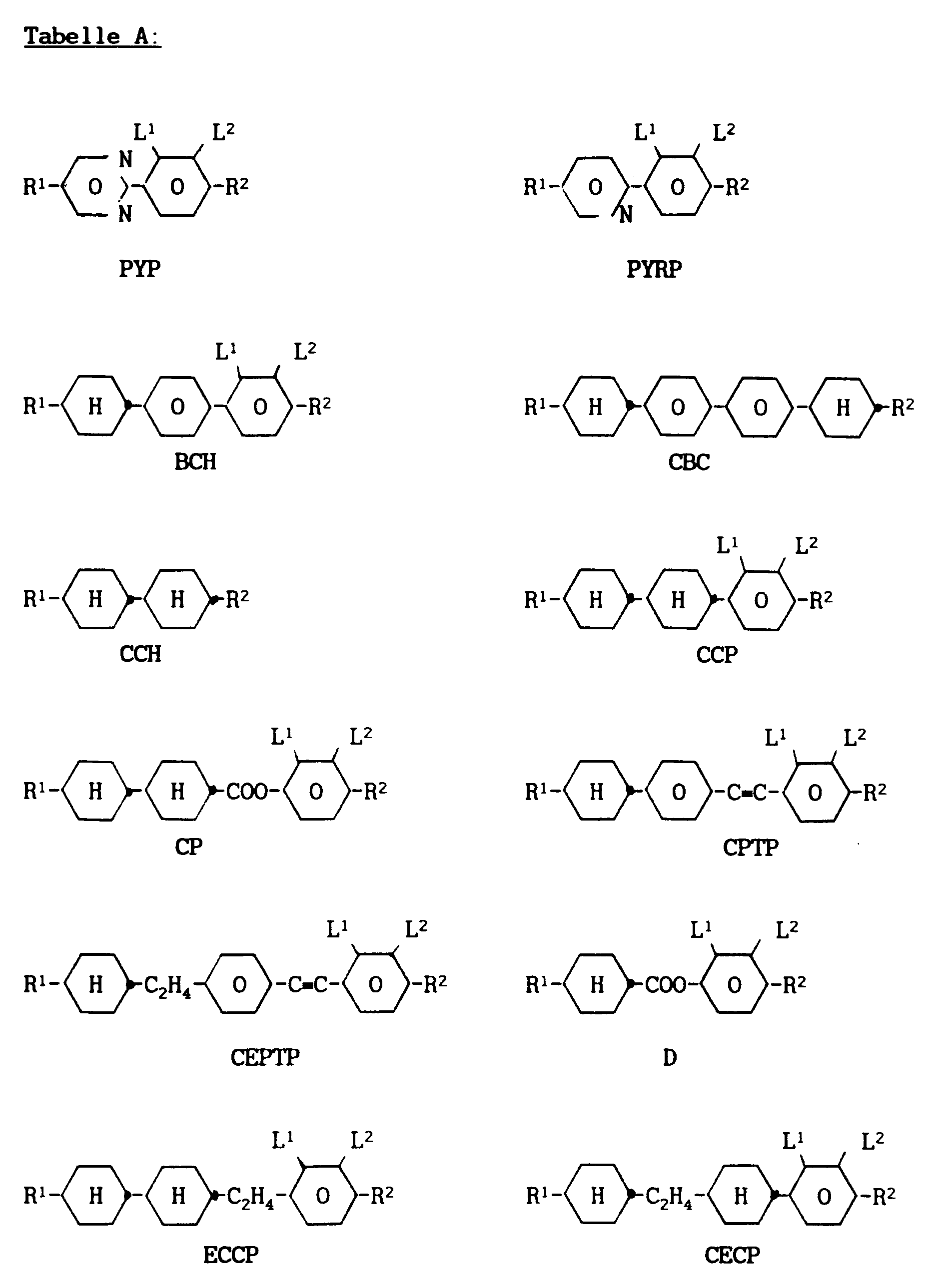

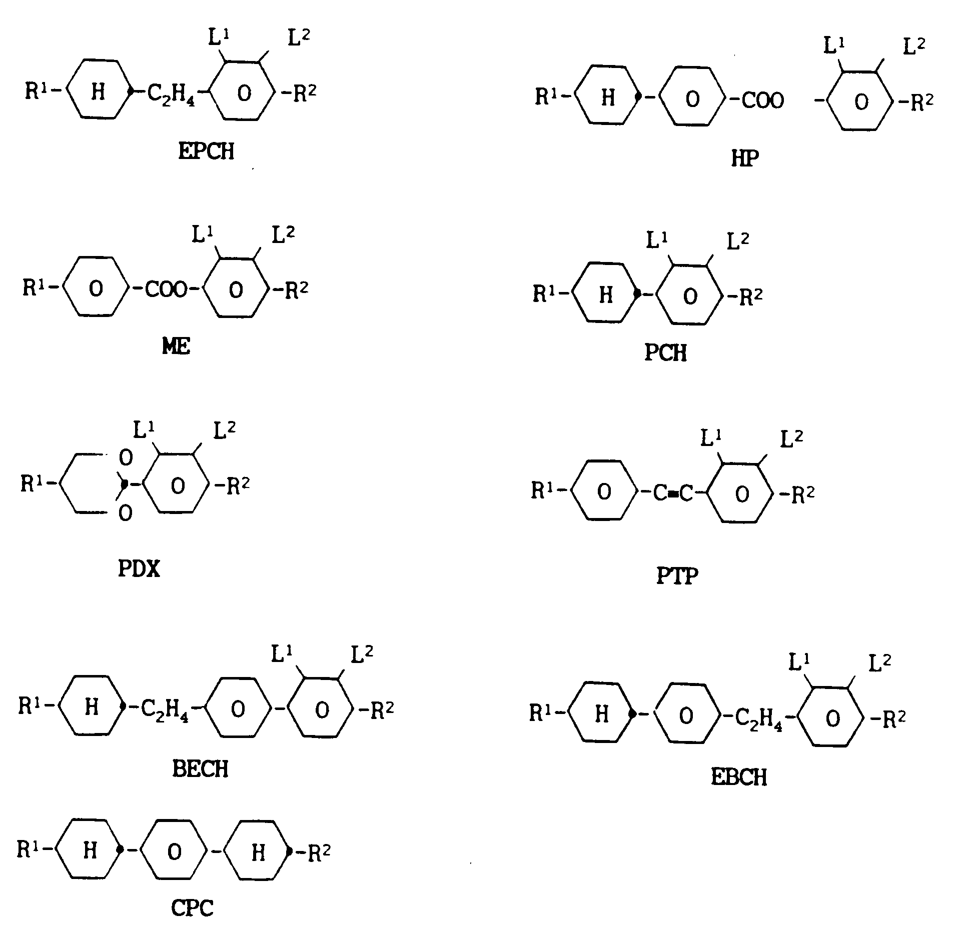

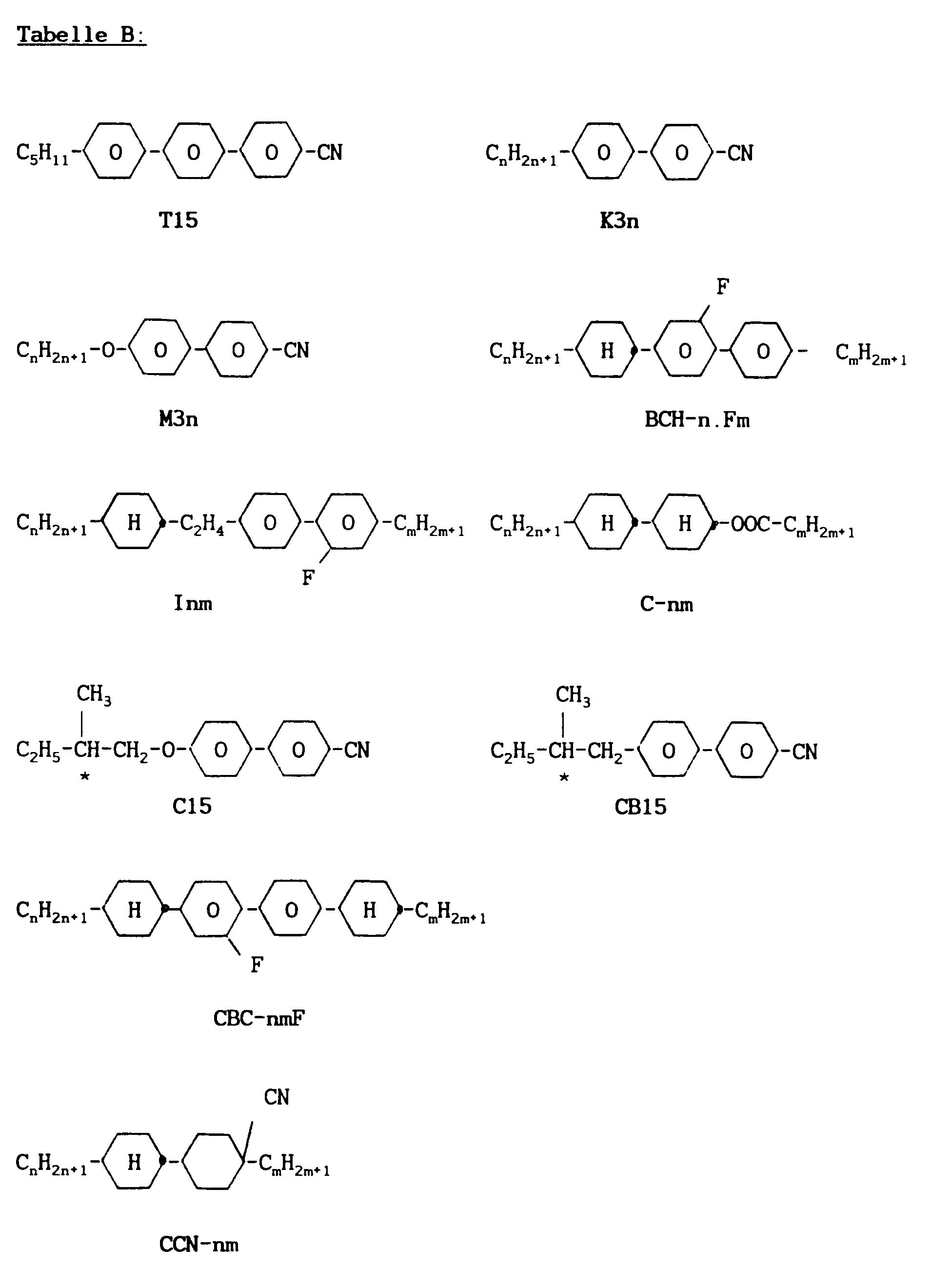

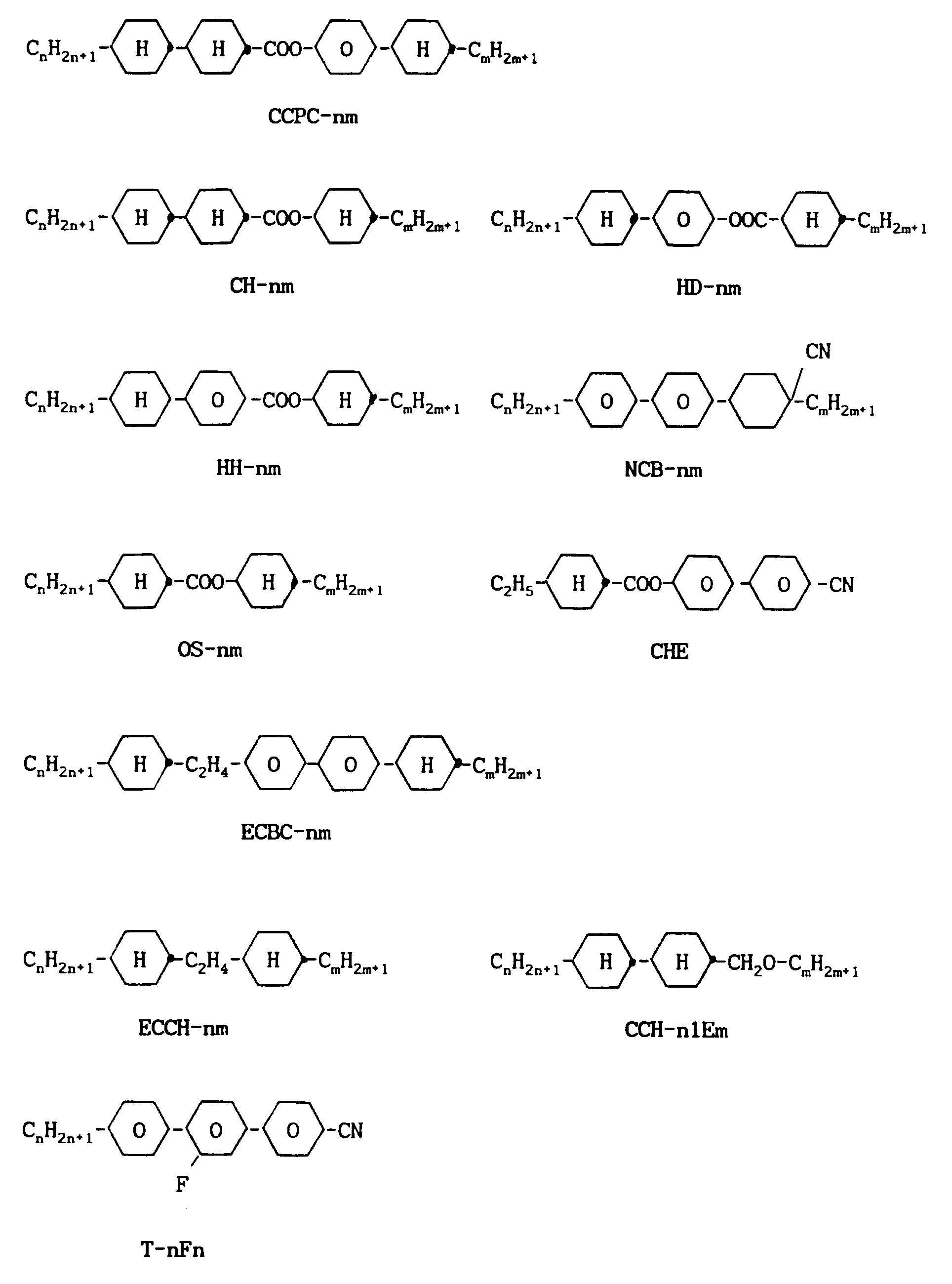

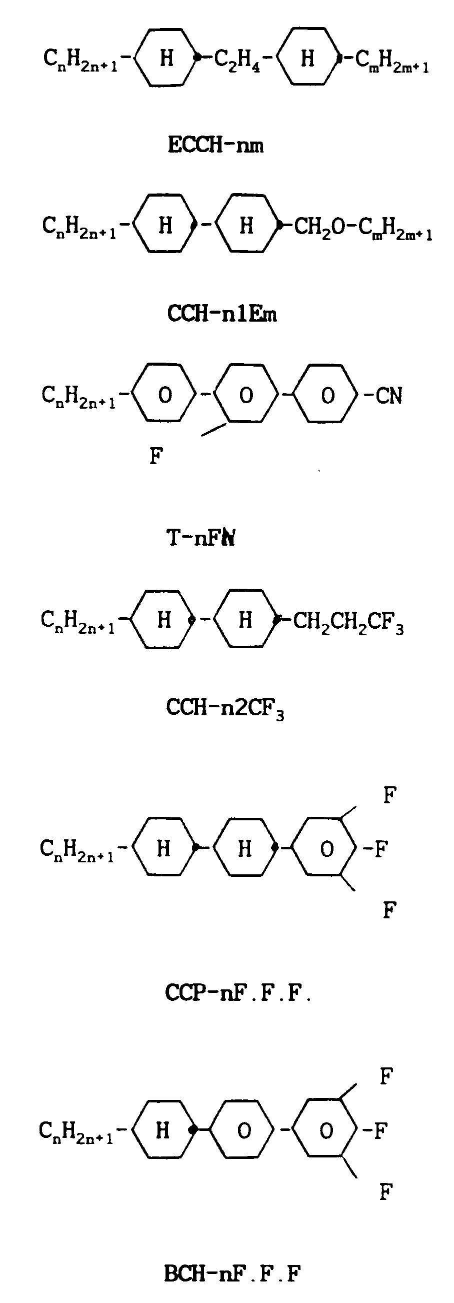

"Übliche Aufarbeitung" bedeutet: man gibt gegebenenfalls Wasser hinzu, extrahiert mit Methylenchlorid, Diethylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- KOT: Kalium-tertiär-butanolat
- THF: Tetrahydrofuran
- pTSOH: p-Toluolsulfonsäure
- TMEDA: Tetramethylethylendiamin

### Beispiel 1

Eine Lösung von 0,1 m 1-trans-4-(trans-4-n-Propylcyclohexyl)-cyclohexyl-2-(3,5-difluorphenyl)-ethan (hergestellt nach Schema 1) und 0,1 m TMEDA in 300 ml THF wird bei ca. -65° mit 0,1 m n-BuLi (1,5 M in Hexan) tropfenweise versetzt. Man rührt noch 30 min bei dieser Temperatur und tropft hinzu bei -60 bis -70 °C 0,1 m B(OCH₃)₃. Man rührt noch 1/2 h nach. Dann tropft man 0,3 m H₂O₂ (30 %ig) zu, wobei die Temperatur +40 °C nicht übersteigen sollte. Nach extraktiver Aufarbeitung erhält man das Phenol, welches durch Kristallisation oder Destillation gereinigt werden kann. Aus diesem Phenol erhält man OCHF₂-Derivat durch Einleiten von CHClF₂ in die THF-Lösung des Phenolats. Nach extraktiver Aufarbeitung und üblicher Reinigung erhält man 1-[trans-4-(trans-4-n-Propylcyclohexyl)-cyclohexyl]-2-(4-difluormethoxy-3,5-difluorphenyl)-ethan.

### Beispiel 2 bis 23:

Analog erhält man aus den entsprechenden Vorstufen der Formel II die folgenden erfindungsgemäßen Verbindungen:

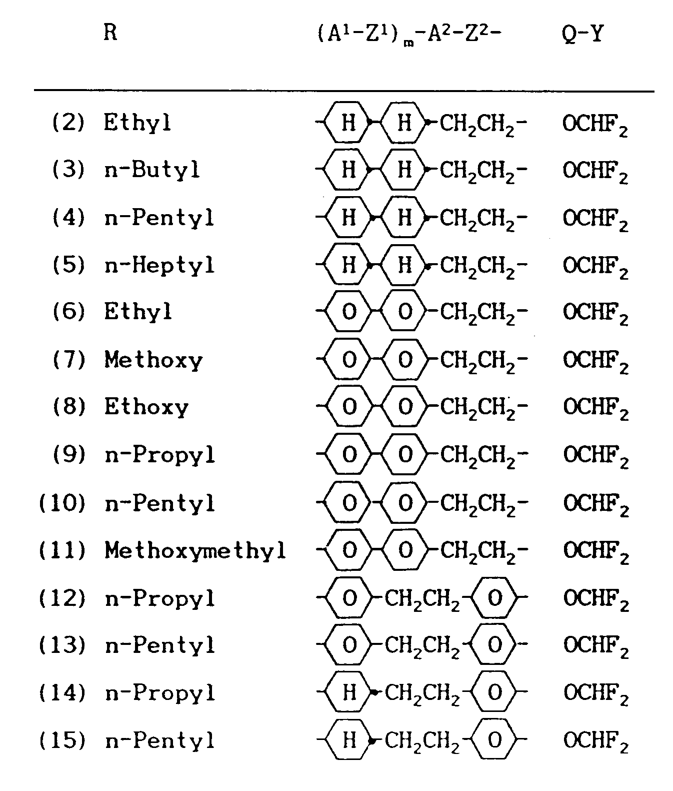

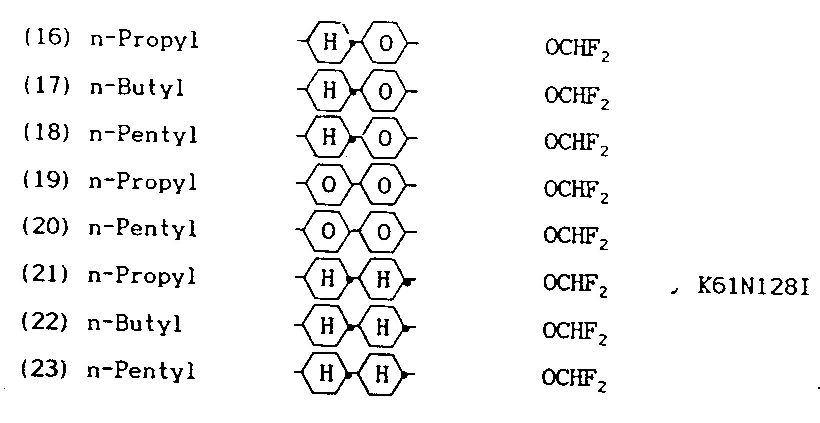

### Beispiel 24

In einem Autoklaven, der auf 0° gekühlt ist, füllt man 2 mol wasserfreie Flußsäure. Dann gibt man eine Mischung aus 0,18 mol Tetrachlormethan und 0,06 mol 1-[trans-4-(trans-4-n-Propylhexyl)-cyclohexyl]-2-(4-hydroxy-3,5-difluorphenyl)-ethan (Beispiel 1) zu. Die Mischung wird ca. 8 Stunden bei 150° gerührt, abgekühlt, auf Eiswasser gegossen und mit Ether nachgewaschen. Die beiden Phasen werden ca. 30 Minuten gerührt, getrennt und die Etherlösung mit 5%iger KOH zur alkalischen Reaktion gewaschen. Nach Trocknen, Abfiltrieren, Abdestillieren und Reinigung erhält man 1-[trans-4-(trans-4-n-Propylcyclohexyl)-cyclohexyl]-2-(4-trifluormethoxy-3,5-difluorphenyl)-ethan.

### Beispiel 25 bis 45:

Analog erhält man aus den entsprechenden Vorstufen der Formel II die folgenden Verbindungen:

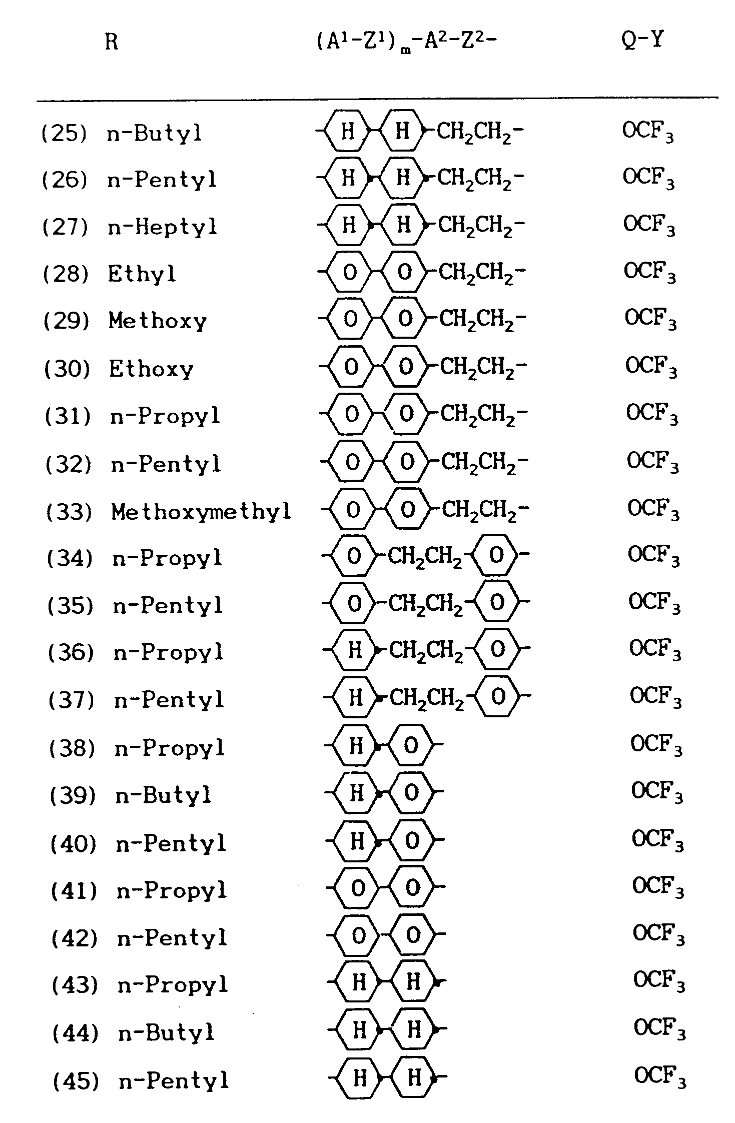

### Beispiel 46

In einem Hastelloy-Autoklaven werden 31,6 g 2-Fluor-4-[trans-4-(trans-4-n-propylcyclohexyl)-cyclohexyl)-phenol (hergestellt aus dem entsprechenden Benzylether durch Hydrierung, der durch Kreuzkopplung von Bis-[trans-4-(trans-4-n-propylcyclohexyl)-cyclohexyl]-zink mit 4-Brom-2-fluorbenzyloxybenzol erhalten wurde) und 49,3 g Tetrachlorkohlenstoff vorgelegt und mit Trockeneis/Aceton gekühlt.

Nach Anlegen eines Vakuums wird mit 66,7 g HF und 1,67 g BF₃ versetzt. Nach achtstündigem Rühren bei 150 °C läßt man auf Zimmertemperatur abkühlen, zieht das HF durch einen Aspirator ab. Der Rückstand wird in Methylenchlorid aufgenommen und mit NaF versetzt um restliches HF abzutrennen. Nach dem Filtrieren wird der Eindampfrückstand durch Chromatographie über Kieselgel und mehrfache Kristallisation aus Hexan und Ethanol aufgereinigt. Man erhält 2-Fluor-4-[trans-4-(trans-4-n-propylcyclohexyl)-cyclohexyl)-trifluormethoxybenzol.

### Beispiel 47 bis 68:

Analog erhält man aus den entsprechenden Phenolen die folgenden Verbindungen:

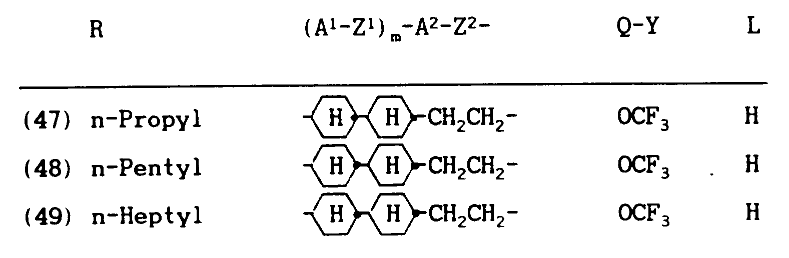

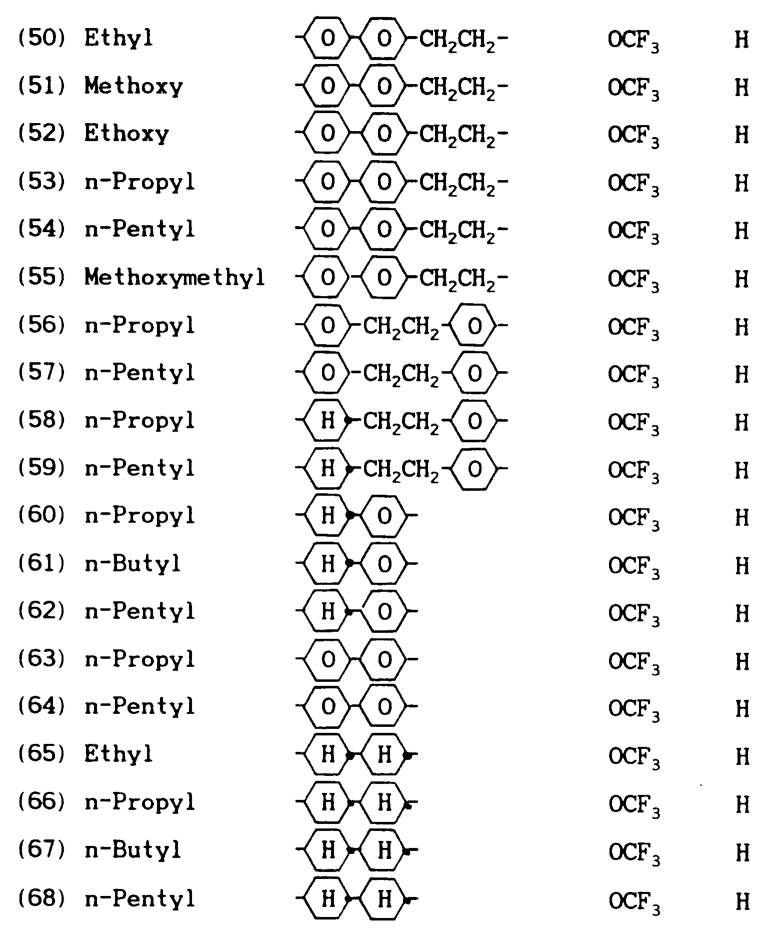

### Beispiel 69 bis 91:

Analog Beispiel 1 erhält man aus den entsprechenden Phenolen die folgenden Verbindungen:

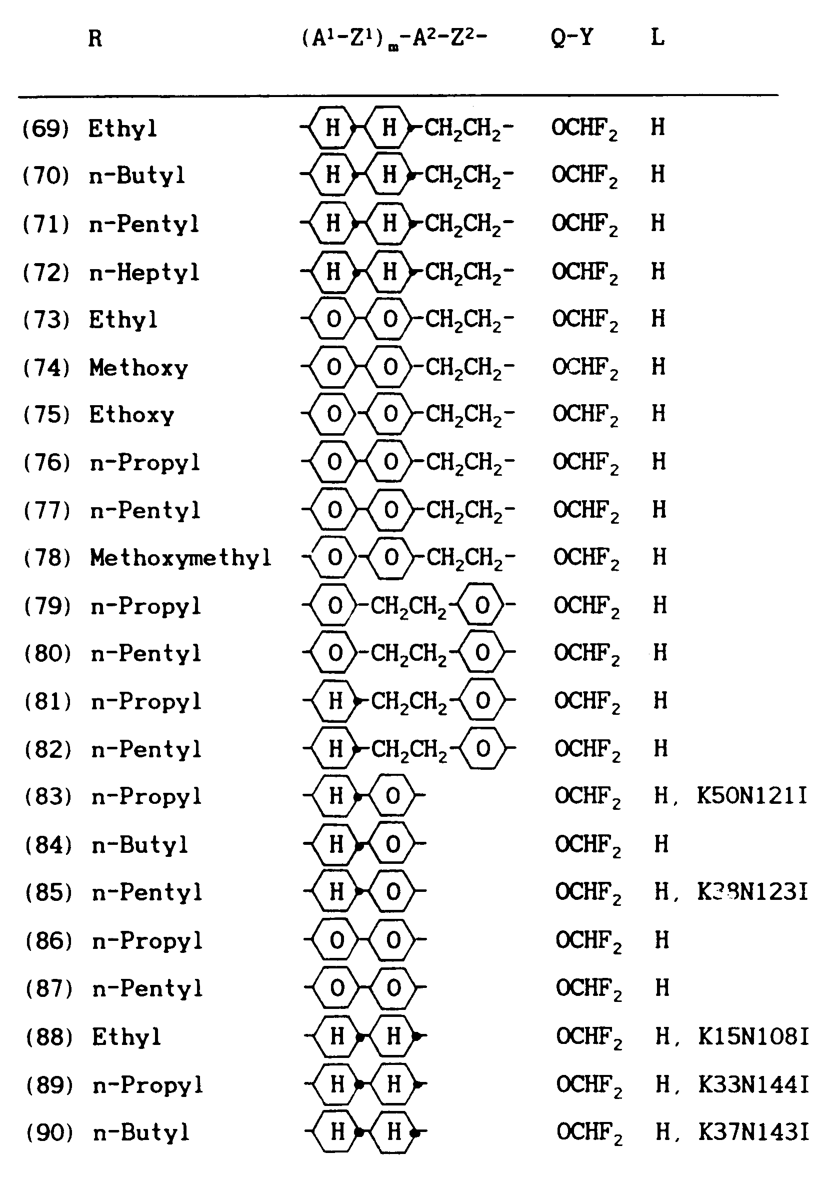

### Beispiel 46

Eine Lösung von 0,1 m 1-[trans-4-(trans-4-n-Propylcyclohexyl)-cyclohexyl]-2-(3,5-difluorphenyl)-ethan (hergestellt nach Schema 3) und 0,1 m TMEDA in 300 ml THF wird bei ca. -65° mit 0,1 m n-BuLi (1,5 M in Hexan) tropfenweise versetzt, Man rührt noch 30 Min. bei dieser Temperatur und setzt dann 0,2 m N-Chlorsuccinimid in 70 ml THF langsam zu. Nach beendeter Zugabe läßt man auf -20° erwärmen und hydrolysiert mit H₂O. Durch Zugabe von Diethylether wird das Produkt vollständig in Lösung gebracht. Nach extraktiver Aufarbeitung und Reinigung durch Chromatographie und Kristallisation erhält man 1-[trans-4-(trans-4-n-Propylcyclohexyl)-cyclohexyl]-2-(4-chlor-3,5-difluorphenyl)-ethan, K 88 N 129 I

### Beispiel 47 bis 143

Analog erhält man aus den entsprechenden Vorstufen der Formel II die folgenden Verbindungen der Formel I bzw. I'':

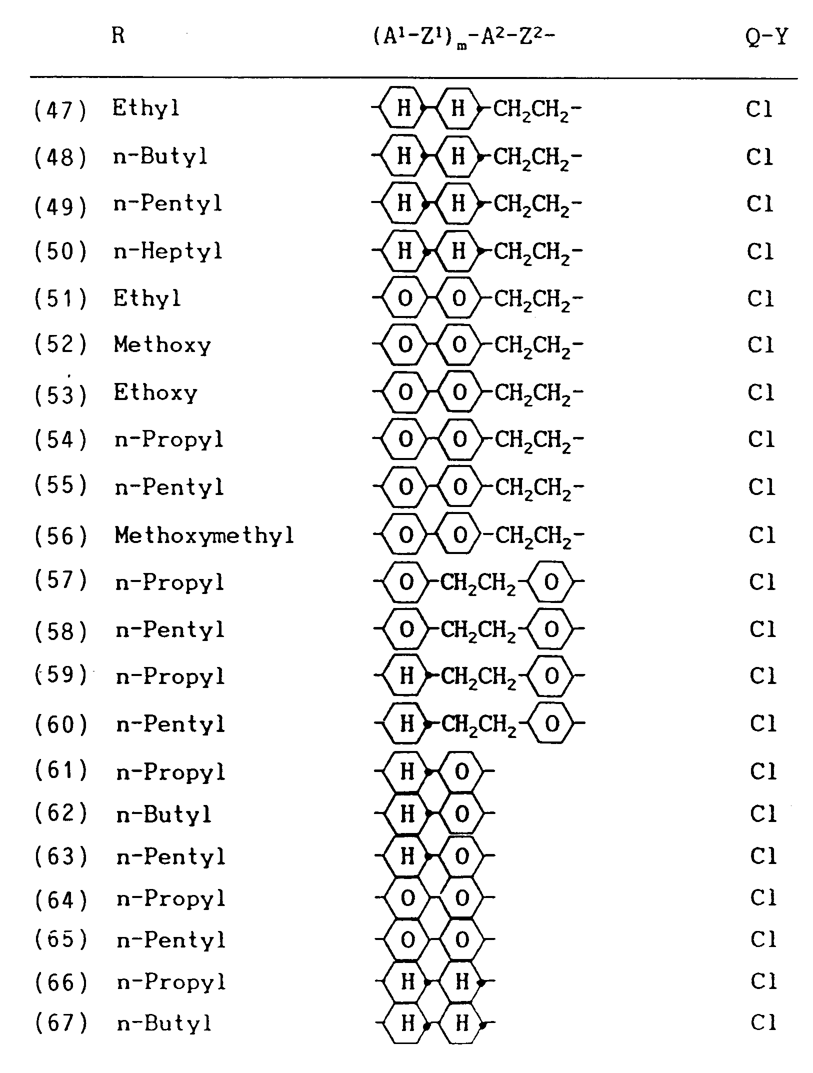

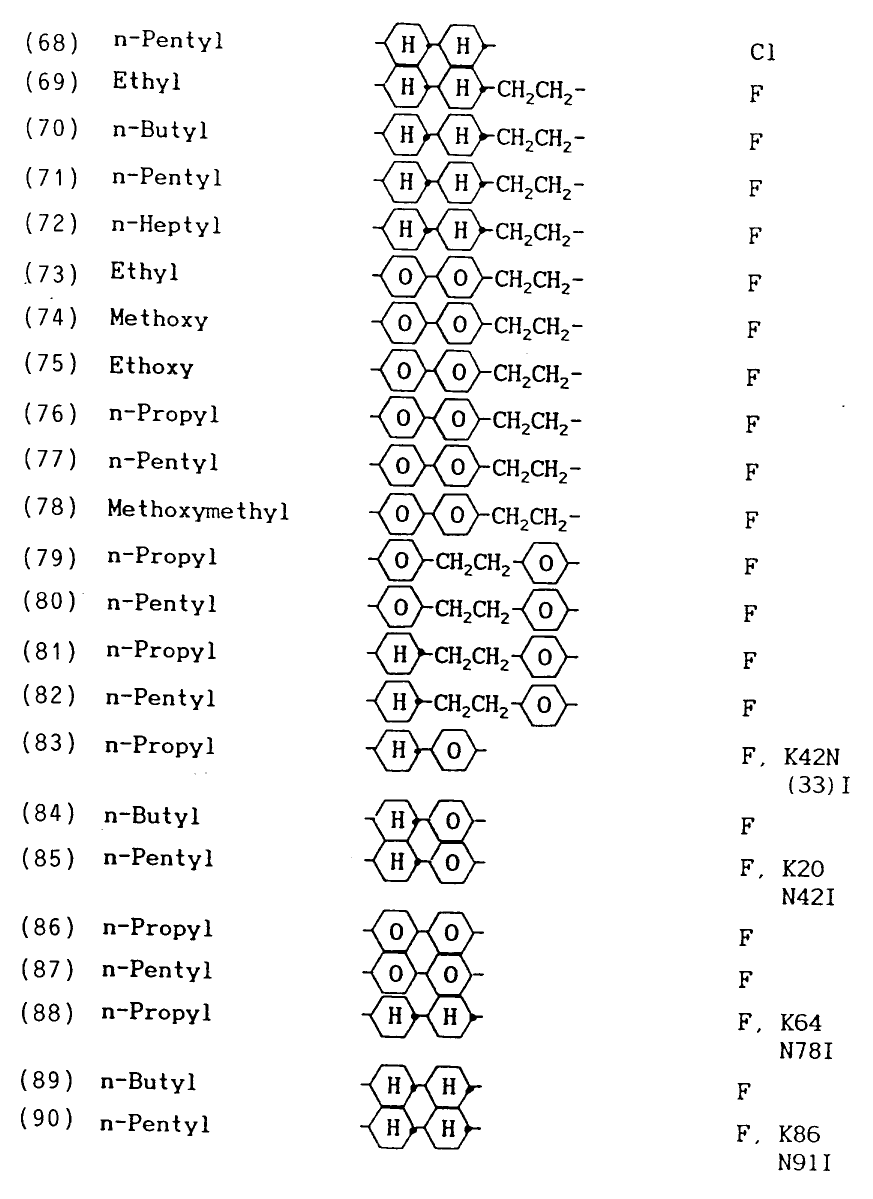

### Beispiel 144

Ein Gemisch von 0,1 m 4-(trans-4-n-Pentylcyclohexyl)-2-fluorbenzoesäure und 0,3 m SF₄ wird im Autoklaven 8 Stunden auf 130° erhitzt. Das erhaltene Rohprodukt wird in Pentan aufgenommen und extraktiv aufgearbeitet. Nach üblicher Aufreinigung durch Destillation und Kristallisation erhält man 4-(trans-4-n-Pentylcyclohexyl)-2-fluortrifluortoluol.

### Beispiel 145

Eine Lösung von 0,01 m 4-(trans-4-n-Propylcyclohexyl)-4'-trifluormethylbiphenyl in 10 ml n-Hexan wird mit 0,01 m TMEDA versetzt. Bei 0-5 °C setzt man 0,01 m n-BuLi zu und rührt noch 1/2 h bei RT und 1/2 h bei 40 °C nach. Dann kühlt man auf 0 °C ab, setzt 20 ml THF zu und tropft bei dieser Temperatur gemäß Schema 21 das Fluorierungsmittel 1-Fluor-2,4,6-trimethylpyridinium Triflat (in THF) zu. Nach extraktiver Aufarbeitung erhält man wie üblich durch Chromatographie und Kristallisation 4-(trans-4-n-Propylcyclohexyl)-3'-fluor-4'-trifluormethylbiphenyl.

### Beispiel 146 bis 175:

Analog Beispiel 144 bzw. 145 erhält man aus den entsprechenden Vorprodukten die folgenden Verbindungen:

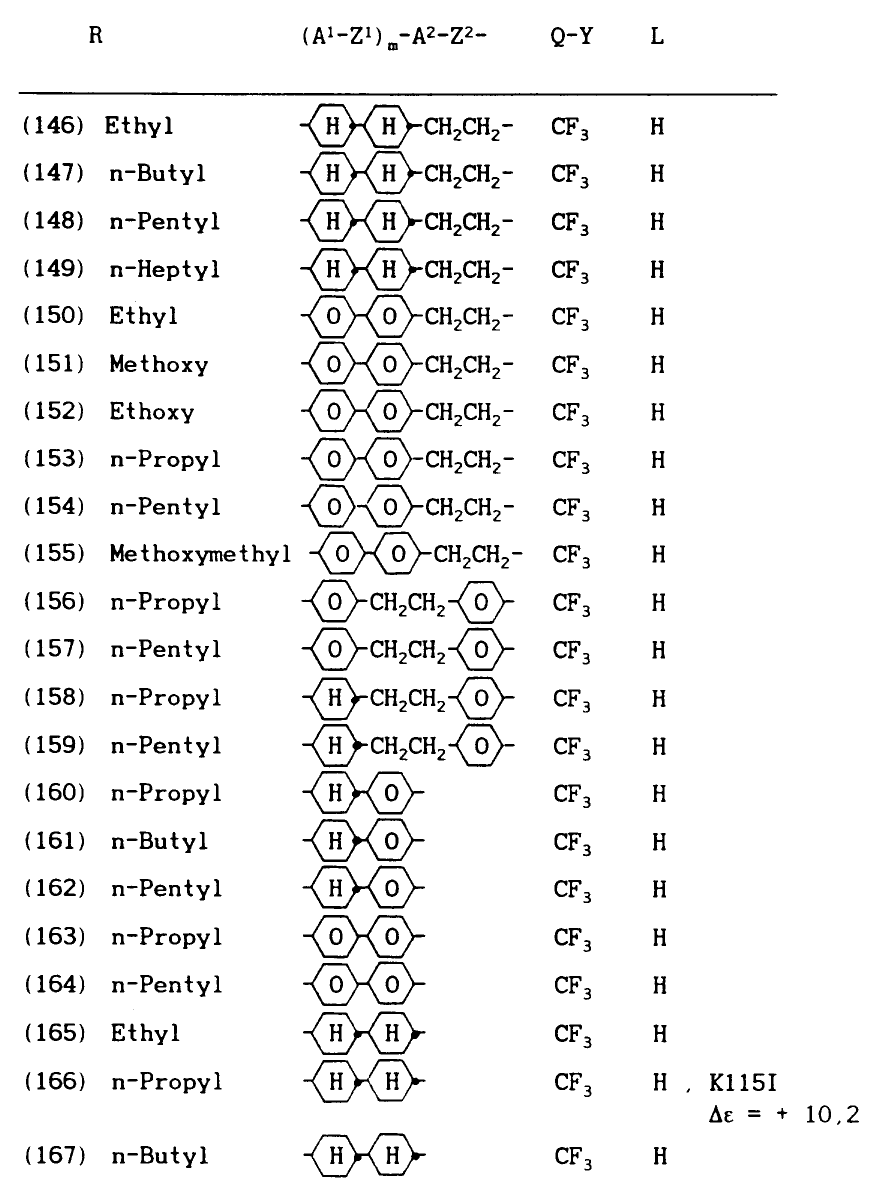

### Beispiel 91

Ein Gemisch von 0,025 m CuJ, 0,0125 m CF₃COONa und 0,0125 m 1-[trans-4-(trans-4-n-Propylcyclohexyl)-cyclohexyl]-2-(4-jod-3,5-difluorphenyl)-ethan [erhältlich gemäß Schema 12] wird in 100 ml N-Methylpyrrolidon unter Rühren auf 150° erhitzt. Nach einer Stunde arbeitet man wie üblich extraktiv auf und erhält nach chromatographischer Aufreinigung 1-[trans-4-(trans-4-n-Propylcyclohexyl)-cyclohexyl]-2-(4-trifluormethyl-3,5-difluorphenyl)-ethan.

### Beispiel 92 bis 121

Analog Beispiel 91 erhält man aus den entsprechenden 3,5-Difluorphenyl-Verbindungen die folgenden Verbindungen:

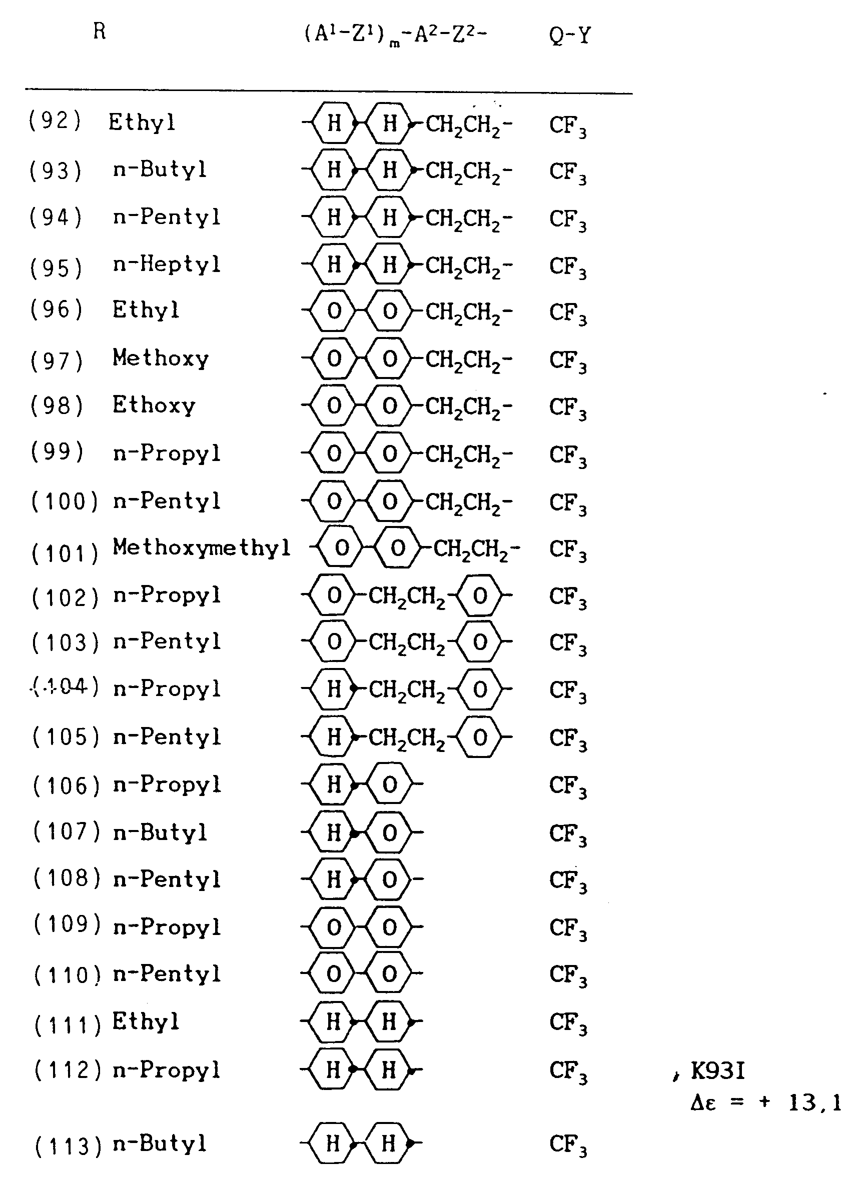

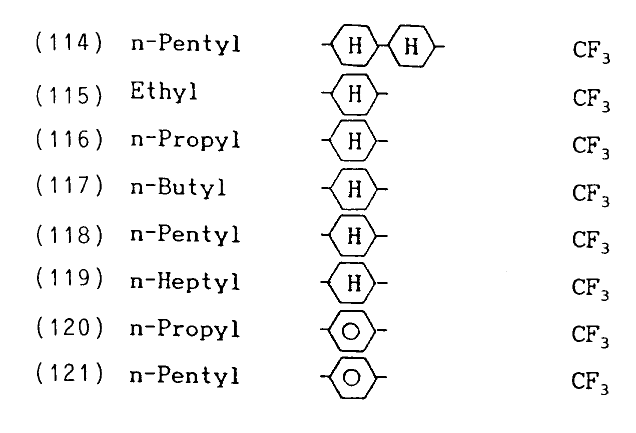

### Beispiel 122

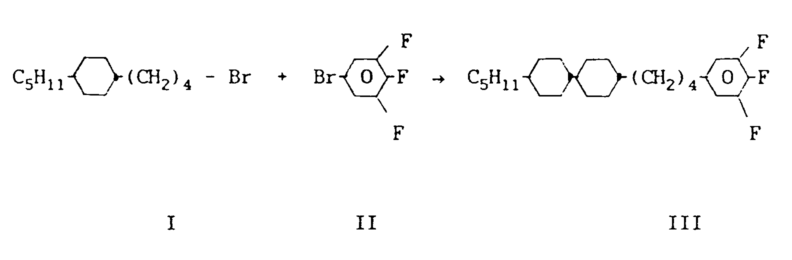

37,1 g I (0,1 Mol) werden in 150 ml eines Lösungsmittelgemisches von THF/Toluol (1 : 4 - Volumenverhältnis) vorgelegt, dann 11,5 g Zinkbromid wasserfrei und darauf 1,4 g Lithiumgranulat hinzugefügt. Das Gemisch wird unter Argon und Rühren 4 Std. zwischen 0 °C und 10 °C mit Ultraschall behandelt, um I in die entsprechende Dialkylzinkverbindung zu überführen. Die zinkorganische Verbindung wird mit 21,1 g II (0,1 Mol) und 1,5 g (2 mol %) 1,1'-Bis(diphenylphosphino)-ferrocen-palladium(II)dichlorid (PdCl₂ dppf) versetzt und nach Entfernung des Ultraschallbades und der Kühlung 24 h bei Zimmertemperatur gerührt. Es wird mit 100 ml gesättigter NH₄Cl-Lösung unter Rühren zersetzt, die organische Phase abgetrennt, die wäßrige Phase 2 x mit Toluol extrahiert. Die vereinigten organischen Extrakte liefern nach dem Trocknen, Einengen und Chromatografieren über Kieselgel mit Hexan III. (I ist herstellbar durch Kettenverlängerung von 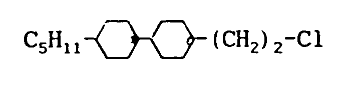
mittels Malonester.) Mit II lassen sich analog I die nachfolgend aufgeführten Alkylbromide umsetzen:

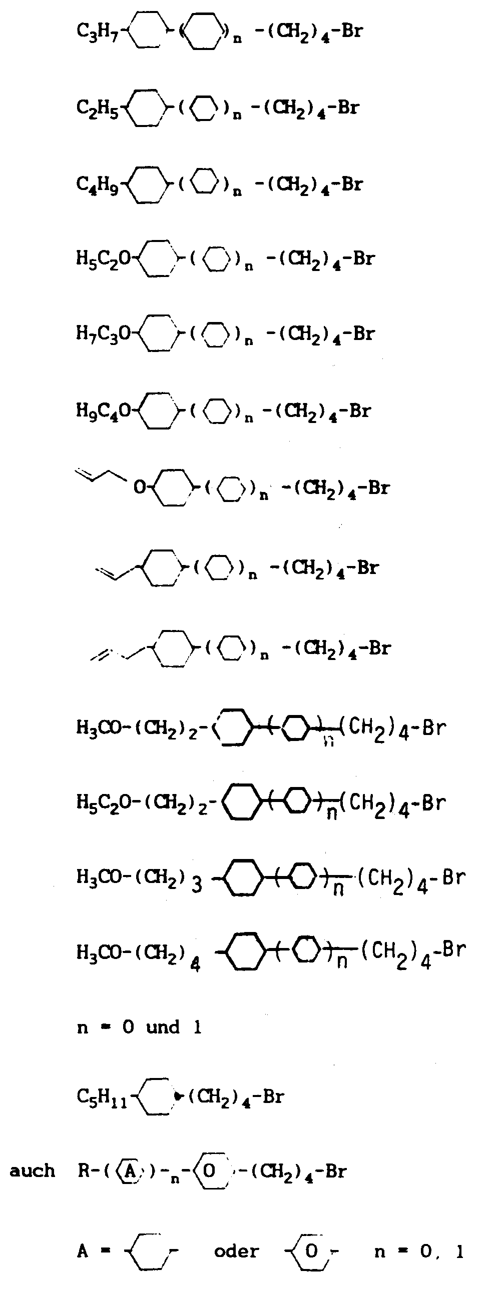

### Beispiel 123

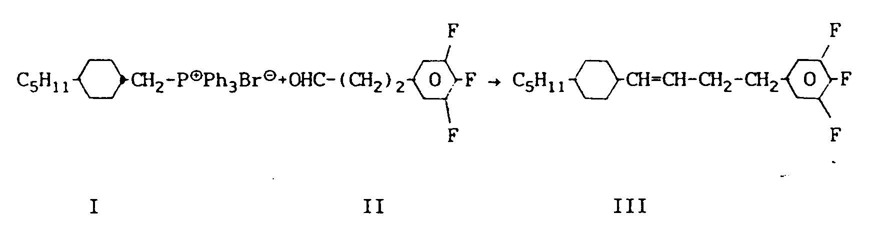

50,9 g (0,1 Mol) Wittigsalz I und 17,2 g Aldehyd II (hergestellt durch Heck-Reaktion von 1-Bromtrifluorbenzol mit Allylalkohol) werden mit 11,5 g Kalium-tert-butylat zwischen 0 °C und 10 °C portionsweise versetzt. Nach der Zugabe wird 24 Std. bei Zimmertemperatur gerührt, auf Wasser gegossen, neutralisiert, mit Toluol extrahiert und der Toluolextrakt nach dem Trocknen eingedampft und über Kieselgel mit Hexan filtriert. Es werden 28 g III erhalten:

### Beispiel 124

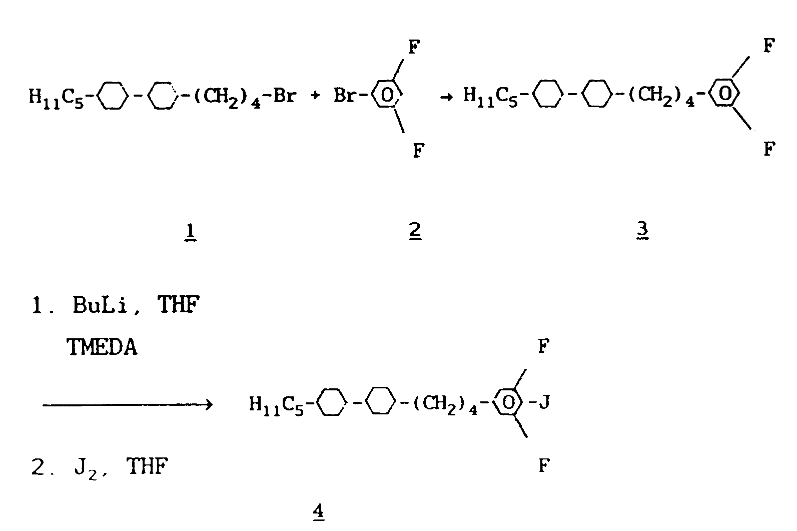

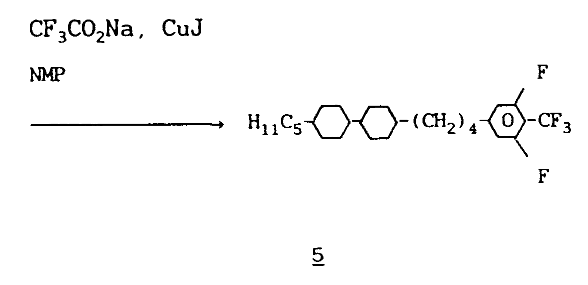

37,1 g (100 mmol) 1 werden analog zum Beispiel 122 durch Umsetzung mit 2 in 3 überführt.

Zu einem Gemisch aus 19,0 g (47 mmol) 3, 7,5 ml TMEDA (50 mmol) und 150 ml THF werden bei -65 bis -70 °C 31 ml n-Buli (15 % in Hexan) zugetropft und es wird 1 Stunde bei -70 °C nachgerührt. Dann wird bei -65 bis -70 °C eine Lösung von 12,0 g (47 mmol) Jod in 25 ml THF zugetropft und 0,5 h bei -70 °C nachgerührt. Man erwärmt auf -30 °C, hydrolysiert mit 15 ml Wasser und reduziert überschüssiges Jod durch Zusatz von 15 ml Natriumhydrogensulfitlösung. Nach üblicher Aufarbeitung und Umkristallisation aus Hexan erhält man 23,9 g (45 mmol) 4. Aus einem Gemisch von 20,2 g (38 mmol) 4, 4,4 g (76 mmol) KF, 22,8 g (168 mmol) Natriumtrifluoracetat und 800 ml NMP werden bei 70 °C und 4 mbar 400 ml NMP abdestilliert. Dann gibt man 14,4 g (76 mmol) getrocknetes CuJ zum Reaktionsgemisch und rührt 5 h bei 160 °C. Anschließend werden ca. 300 ml NMP abdestilliert. Man läßt auf RT abkühlen und versetzt mit 300 ml MTB-Ether. Man wäscht mit Wasser, trocknet mit Na₂SO₄, filtriert und engt zum Rückstand ein. Nach Chromatographie an Kieselgel mit Hexan erhält man 5.

### Beispiel 210

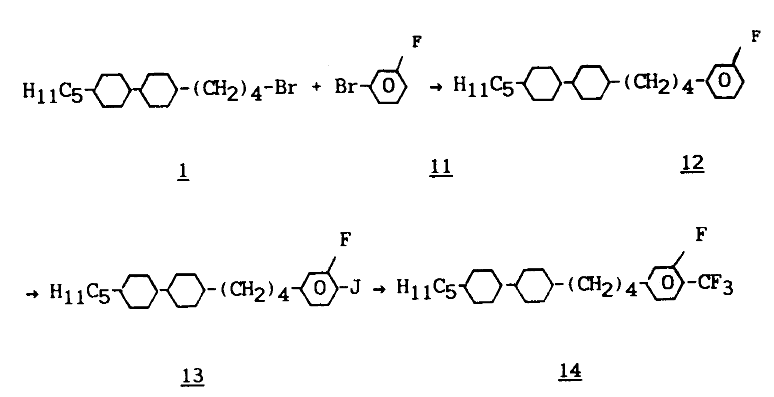

31,1 g (100 mmol) 1 werden analog zum Beispiel 207 durch Umsetzung mit 11 in 12 überführt.

Zu einem Gemisch aus 18,2 g (47 mmol) 12, 7,4 g Kaliumtertiärbutylat und 110 ml THF werden bei -100 °C 40 ml n-BuLi zugetropft und es wird 1 h bei -100 °C nachgerührt. Anschließend wird bei -85 bis -90 °C eine Lösung von 15,9 g Jod in 60 ml THF zugetropft. Man rührt noch 0,5 h bei -90 °C nach, erwärmt auf -30 °C, hydrolysiert mit 30 ml Wasser, säuert mit konz. Salzsäure an und reduziert überschüssiges Jod durch Zusatz von Natriumhydrogensulfitlösung. Nach üblicher Aufarbeitung und Umkristallisation aus Hexan erhält man 21,4 g (41 mmol) 13. 19,5 g (38 mmol) 13 werden entsprechend Beispiel 209 durch Umsatz mit Natriumtrifluoracetat in 14 überführt. Man erhält nach chromatographischer Aufreinigung 14.

### Beispiel 211

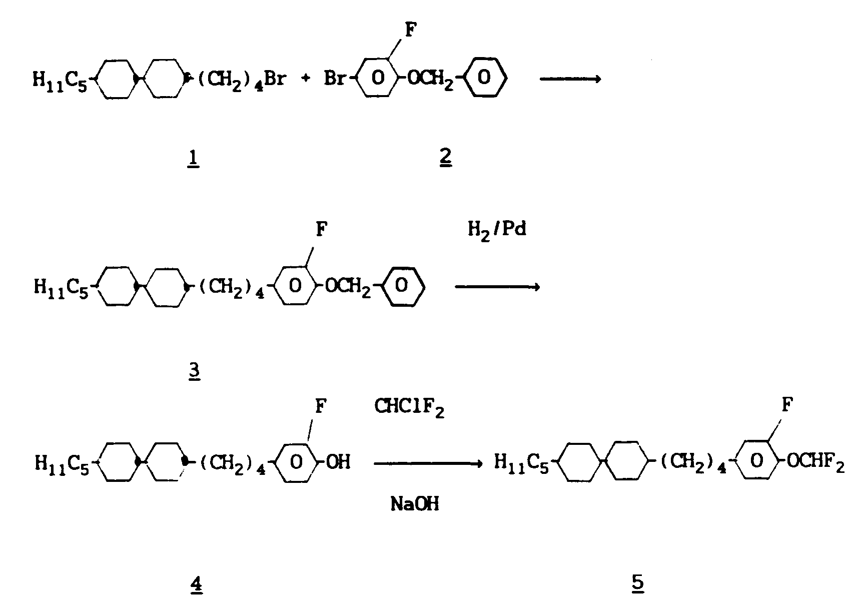

Gemäß obenstehendem Syntheseschema wird analog Beispiel 207 nach Überführung von 1 in die zinkorganische Verbindung durch eine Pd-(II)-katalysierte Kopplungsreaktion mit 2 die Verbindung 3 erhalten. Hydrogenolytische Spaltung des Benzylethers führt zum Phenol 4.

4,0 g (0,01 mol) dieses Phenols werden in THF vorgelegt, 3,1 g 32 %ige Natronlauge und 0,5 g Tetrabutylammoniumhydrogensulfat zugegeben, auf 50 °C erwärmt und unter Rühren solange Chlordifluormethan eingeleitet, bis dieses an einem mit Trockeneis gekühlten Kühler kondensiert. Nach dem Abkühlen wird die THF-Lösung von dem ausgefallenen schmierigen Produkt abdekantiert, eingeengt und das erhaltene 5 aus Ethanol umkristallisiert.

### Beispiel 125

Zu einer Lösung von 15 g 4-(trans-4-n-Propyl-cyclohexyl)-2-fluor-4'-brombiphenyl in 50 ml THF tropft man bei -70 °C 25 ml Butyllithium (15 % in Hexan) und gibt nach 30 min eine gekühlte Lösung von 4,5 g ZnBr₂ in 25 ml THF zu. Nach Zugabe von 8,5 g 1-Brom-3,4,5-trifluorbenzol in 75 ml THF und 0,6 g PdCl₂ - dppf läßt man auf Raumtemperatur kommen. Nach 24 h Rühren gießt man in 100 ml gesättigte NH₄Cl-Lösung und arbeitet wie üblich auf. Man erhält 4''-(trans-4-n-Propylcyclohexyl)-2'',3,4,5-tetrafluorterphenyl, K 148 N 233 I.

Analog erhält man aus den entsprechenden Bromaromaten:
4''-n-Propyl-2'',3,4,5-tetrafluorterphenyl
4''-n-Pentyl-2'',3,4,5-tetrafluorterphenyl
4'-(trans-4-methoxymethylcyclohexyl)-3,4,5-trifluorbiphenyl

### Beispiel 126

Ein Gemisch von 10,5 g 1-Brom-3,4,5-trifluorbenzol, 7,4 g p-Ethoxystyrol, 0,25 g Pd-acetat, 0,6 g Tri-o-tolylphosphin, 7 g Triethylamin und 125 ml Acetonitril wird am Rückfluß erhitzt bis zur Beendigung der Reaktion. Nach üblicher Aufarbeitung erhält man 1-(p-Ethoxyphenyl)-2-(3,4,5-trifluorphenyl)-ethen (K 76 I), welches an Pd-C (5 %) in THF zu 1-(p-Ethoxyphenyl)-2-(3,4,5-trifluorphenyl)-ethan hydriert wird, K 45 I.

### Beispiel 214

Aus p-Ethoxystyrol und dem Benzylether des p-Brom-o-fluorphenols erhält man analog Beispiel 213 1-(p-Ethoxyphenyl)-2-(3-fluor-4-benzyloxyphenyl)-ethen. Durch Hydrieren an Pd-C (5 %) im THF erhält man 1-(p-Ethoxyphenyl)-2-(4-hydroxy-3-fluorphenyl)-ethan, wovon 41,3 g zusammen mit 5,6 g Tetrabutylammoniumhydogensulfat bei 40 °C in 500 ml THF gelöst werden. In diese Lösung werden 43 g Chlordifluormethan so eingeleitet, daß eine geringe Menge am Trockeneisrückflußkühler kondensiert. Dann werden 32 g NaOH (50 %) unter gutem Rühren innerhalb von 10 min zugetropft. Nach einer weiteren Stunde Rühren bei 40° wird abgekühlt und von dem gebildeten schmierigen Niederschlag abdekantiert. Nach Abdampfen des Lösungsmittels wird der Rückstand chromatographisch und durch Umkristallisation aufgereinigt. Man erhält 1-(p-Ethoxyphenyl)-2-(3-fluor-4-difluormethoxyphenyl)-ethan, K 43 I.

### Beispiel 215

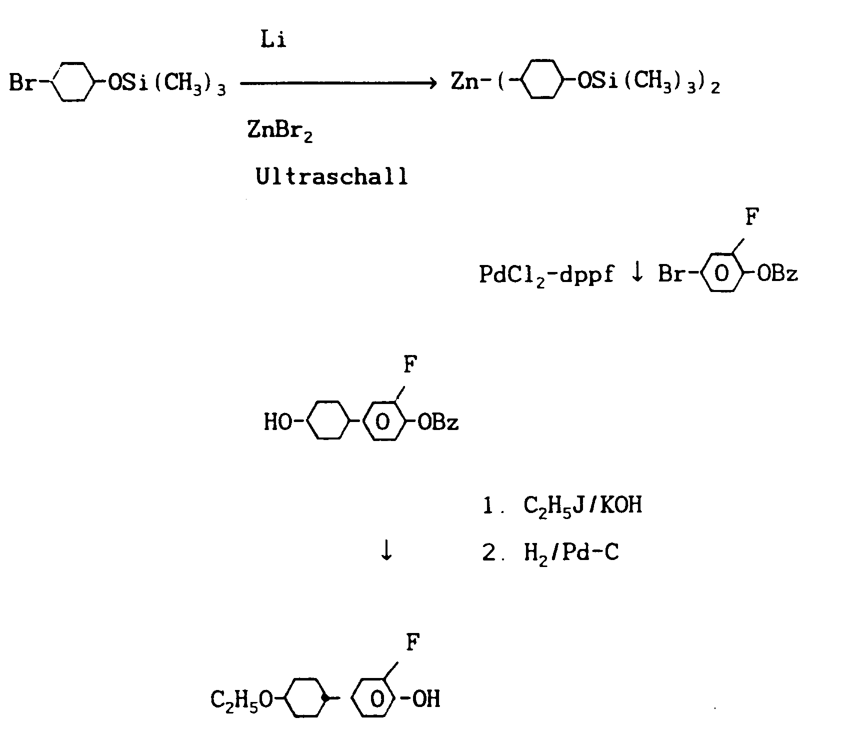

Zu einer Lösung von 8,2 des erhaltenen p-(trans-4-Ethoxycyclohexyl)-o-fluorphenol in 80 ml THF werden 11 g NaOH (32 %) zugegeben und 13,8 g Chlordifluormethan eingeleitet (Trockeneisrückflußkühler). Nach einer Stude nachrühren wird vom Niederschlag abdekantiert und die Lösung zum Rückstand eingeengt. Durch Kugelrohrdestillation erhält man p-(trans-4-Ethoxycyclohexyl)-o-fluordifluormethoxybenzol, Kp₁ ∼ 220 °C, Tg-62°.

### Beispiel A

| | | | |
|---|---|---|---|
| PCH-5F | 10,0 % | S → N [°C] | < -40 |
| PCH-6F | 7,0 % | Klärpunkt [°C] | +64 |
| PCH-7F | 15,0 % | Viskosität 20 °C | 14 |
| CCP-2OCF₃ | 10,0 % | Δn (589 nm, 20 °C) | 0,0800 |
| CCP-3OCF₃ | 12,0 % | n_{ε} (589 nm, 20 °C) | 1,5576 |
| CCP-4OCF₃ | 8,0 % | V_{(10,0,20)} | 1,61 |
| CCP-5OCF₃ | 12,0 % | V_{(50,0,20)} | 2,04 |
| CCP-3F.F.F | 10,0 % | V_{(90,0,20)} | 2,66 |
| BCH-5F.F | 16,0 % | | |

### Beispiel B

| | | | |
|---|---|---|---|
| PCH-5F | 12,0 % | S → N [°C] | < -30 |
| PCH-7F | 8,0 % | Klärpunkt [°C] | +80 |
| CCP-2OCF₃ | 10,0 % | Viskosität 20 °C | - |
| CCP-3OCF₃ | 12,0 % | Δn (589 nm, 20 °C) | +0,0756 |
| CCP-4OCF₃ | 8,0 % | n_{ε} (589 nm, 20 °C) | 1,5489 |
| CCP-5OCF₃ | 12,0 % | V_{(10,0,20)} | 1,66 |
| ECP-3F.F | 12,0 % | V_{(50,0,20)} | 2,12 |
| CCP-3F.F.F | 14,0 % | V_{(90,0,20)} | 2,73 |
| CCP-5F.F.F | 12,0 % | | |

### Beispiel C

| | | | |
|---|---|---|---|
| PCH-5F | 12,0 % | S → N [°C] | - |
| PCH-7F | 7,0 % | Klärpunkt [°C] | +78 |
| CCP-2OCF₃ | 10,0 % | Viskosität 20 °C | 16 |
| CCP-3OCF₃ | 12,0 % | Δn (589 nm, 20 °C) | 0,0844 |
| CCP-4OCF₃ | 8,0 % | n_{ε} (589 nm, 20 °C) | 1,5610 |
| CCP-5OCF₃ | 12,0 % | V_{(10,0,20)} | 1,61 |
| BCH-3F.F | 8,0 % | V_{(50,0,20)} | 2,06 |
| BCH-5F.F | 6,0 % | V_{(90,0,20)} | 2,72 |
| CCP-3F.F.F | 13,0 % | | |
| CCP-5F.F.F | 12,0 % | | |

### Beispiel D

| | | | |
|---|---|---|---|
| PCH-5F | 12,0 % | S → N [°C] | - |
| PCH-7F | 8,0 % | Klärpunkt [°C] | +77 |
| CCP-2OCF₃ | 10,0 % | Viskosität 20 °C | - |
| CCP-3OCF₃ | 12,0 % | Δn (589 nm, 20 °C) | +0,0847 |
| CCP-4OCF₃ | 8,0 % | n_{ε} (589 nm, 20 °C) | 1,5605 |
| CCP-5OCF₃ | 12,0 % | V_{(10,0,20)} | 1,59 |
| BCH-3F.F.F | 14,0 % | V_{(50,0,20)} | - |
| ECCP-3F.F | 12,0 % | V_{(90,0,20)} | - |
| CCP-5F.F.F | 12,0 % | | |

### Beispiel E

| | | | |
|---|---|---|---|
| PCH-5F | 11,0 % | S → N [°C] | - |
| PCH-7F | 9,0 % | Klärpunkt [°C] | +75 |
| CCP-2OCF₃ | 8,0 % | Viskosität 20 °C | - |
| CCP-3OCF₃ | 10,0 % | Δn (589 nm, 20 °C) | 0,0876 |
| CCP-4OCF₃ | 7,0 % | n_{ε} (589 nm, 20 °C) | 1,5666 |
| CCP-5OCF₃ | 10,0 % | V_{(10,0,20)} | 1,51 |
| BCH-3F.F | 10,0 % | V_{(50,0,20)} | 1,95 |
| BCH-5F.F | 10,0 % | V_{(90,0,20)} | 2,54 |
| CCP-3F.F.F | 13,0 % | | |
| CCP-5F.F.F | 12,0 % | | |

### Beispiel F

| | | | |
|---|---|---|---|
| PCH-5F | 12,0 % | S → N [°C] | - |
| PCH-7F | 7,0 % | Klärpunkt [°C] | +76 |
| CCP-2OCF₃ | 10,0 % | Viskosität 20 °C | - |
| CCP-3OCF₃ | 12,0 % | Δn (589 nm, 20 °C) | 0,0835 |
| CCP-4OCF₃ | 8,0 % | n_{ε} (589 nm, 20 °C) | 1,5595 |
| CCP-5OCF₃ | 12,0 % | V_{(10,0,20)} | 1,49 |
| BCH-3F.F.F | 8,0 % | V_{(50,0,20)} | 1,93 |
| BCH-5F.F | 6,0 % | V_{(90,0,20)} | 2,52 |
| CCP-3F.F.F | 13,0 % | | |
| CCP-5F.F.F | 12,0 % | | |

### Beispiel G

| | | | |
|---|---|---|---|
| PCH-5F | 8,0 % | S → N [°C] | - |
| PCH-7F | 4,0 % | Klärpunkt [°C] | +81 |
| CCP-2OCF₃ | 10,0 % | Viskosität 20 °C | - |
| CCP-3OCF₃ | 12,0 % | Δn (589 nm, 20 °C) | +0,0907 |
| CCP-4OCF₃ | 8,0 % | n_{ε} (589 nm, 20 °C) | 1,5672 |
| CCP-5OCF₃ | 12,0 % | V_{(10,0,20)} | 1,46 |
| BCH-3F.F.F | 14,0 % | V_{(50,0,20)} | - |
| BCH-5F.F.F | 10,0 % | V_{(90,0,20)} | - |
| CCP-3F.F.F | 10,0 % | | |
| CCP-5F.F.F | 8,0 % | | |
| ECCP-3F.F | 4,0 % | | |

### Beispiel H

| | | | |
|---|---|---|---|
| PCH-5F | 8,0 % | S → N [°C] | - |
| CCP-2OCF₃ | 10,0 % | Klärpunkt [°C] | +86 |
| CCP-3OCF₃ | 12,0 % | Viskosität 20 °C | - |
| CCP-4OCF₃ | 8,0 % | Δn (589 nm, 20 °C) | +0,0930 |
| CCP-5OCF₃ | 12,0 % | n_{ε} (589 nm, 20 °C) | 1,5697 |
| BCH-3F.F.F | 14,0 % | V_{(10,0,20)} | 1,45 |
| BCH-5F.F.F | 11,0 % | V_{(50,0,20)} | 1,89 |
| CCP-3F.F.F | 12,0 % | V_{(90,0,20)} | 2,46 |
| CCP-5F.F.F | 7,0 % | | |
| ECCP-3F.F | 6,0 % | | |

## Patentansprüche (Patentansprüche für folgende(n) Vertragsstaat(en): AT, BE, CH, LI, DE, FR, GB, IT, LU, NL, SE)

1. Fluorbenzolderivate der Formel I, 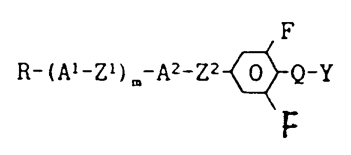 worin
R H, einen unsubstituierten, einen einfach durch CN oder CF₃ oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig von einander durch -O-, -S-, 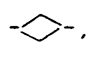 -CO-, -CO-O- , -O-CO- oder -O-CO-O- so ersetzt sein können, daß O-Atome nicht direkt miteinander verknüpft sind,
A¹ und A² jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Fluor substituiert sein können,
Z¹ und Z² jeweils unabhängig voneinander -CO-O-, -O-CO-, -CH₂O-, -OCH₂-, -CH₂CH₂-, -CH=CH-, -C≡C- oder eine Einfachbindung, einer der Reste Z¹ und Z² auch -(CH₂)₄- oder -CH=CH-CH₂CH₂-,
m 0, 1 oder 2,
Y F oder Cl, und
Q eine Einfachbindung, -CF₂-, -OCF₂- oder -OCHF-
bedeutet, mit der Maßgabe, daß folgende Verbindungen ausgeschlossen sind:
(a) 4-Propyl-4'-(3,5-difluor-4-difluormethoxyphenyl)-trans, trans-bicyclohexyl
(b) 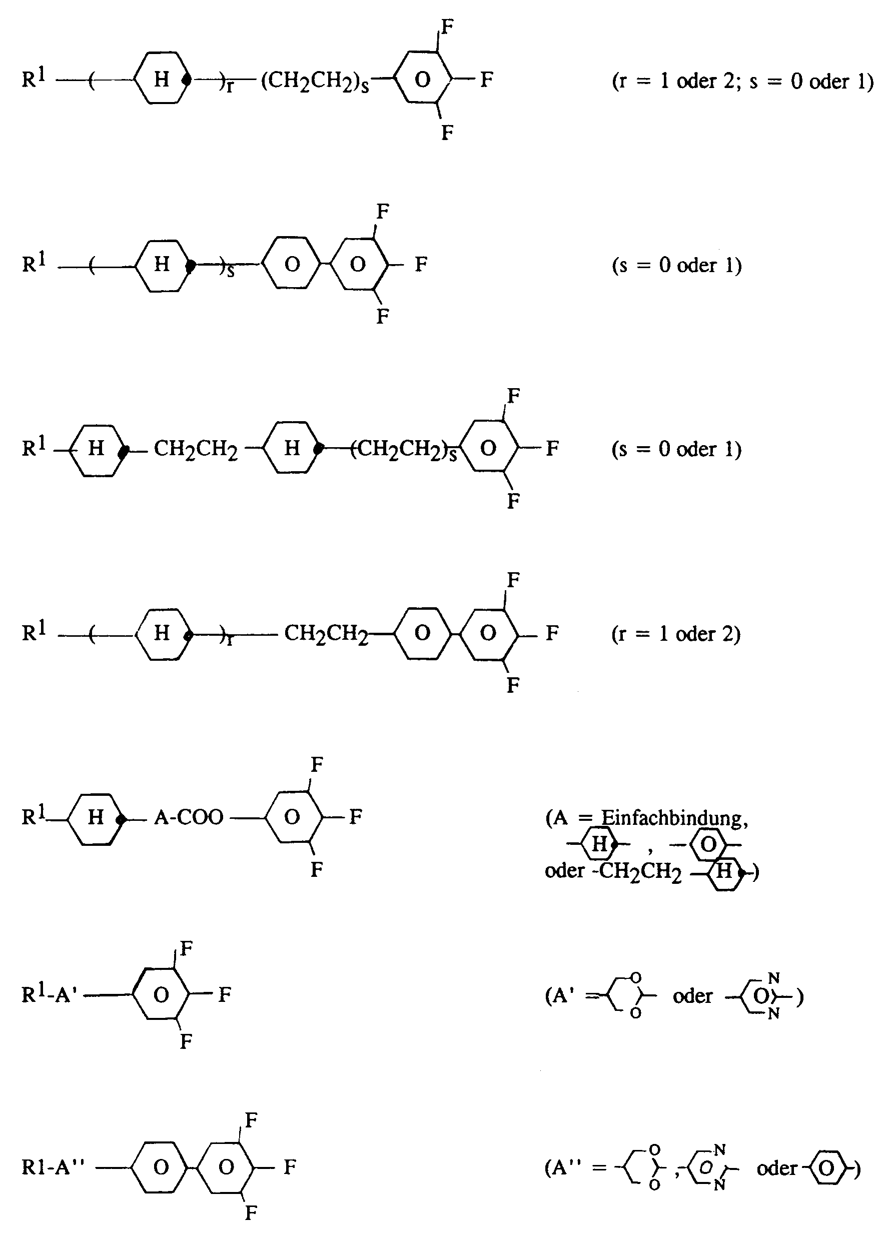
wobei R1 jeweils eine Alkylgruppe mit 1 bis 10 C-Atomen bedeutet.

2. Verwendung von Verbindungen der Formel I als Komponenten flüssigkristalliner Medien.

3. Flüssigkristallines Medium mit mindestens zwei flüssigkristallinen Komponenten, dadurch gekennzeichnet, daß sie mindestens eine Verbindung der Formel I enthält.

4. Flüssigkristallines Medium enthaltend neben einem oder mehreren Fluorbenzolderivaten der Formel I'' 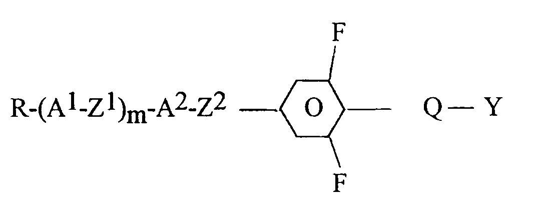 worin
R H, einen unsubstituierten, einen einfach durch CN oder CF₃ oder einen mindestens einfach durch Halogen substituierten Alkyl- oder Alkenylrest mit 1 bis 15 C-Atomen wobei in diesen Resten auch eine oder mehrere CH₂-Gruppen jeweils unabhängig voneinander durch -O-, -S-, 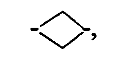 -CO-, -CO-O-, -O-CO- oder -O-CO-O- so ersetzt sein können, daß O-Atome nicht direkt miteinander verknüpft sind,
A¹ und A² jeweils unabhängig voneinander einen
(a) trans-1,4-Cyclohexylenrest, worin auch eine oder mehrere nicht benachbarte CH₂-Gruppen durch -O- und/oder -S-ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo (2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Rest (a) und (b) durch CN oder Fluor substituiert sein können,
Z¹ und Z² jeweils unabhängig voneinander -CO-O-, -O-CO-, -CH₂O-, -OCH₂-, -CH₂CH₂-, -CH=CH.-, -C≡C- oder eine Einfachbindung, einer der Reste Z1 und Z2 auch -(CH₂)₄- oder -CH=CH-CH₂CH₂-,
m 0, 1 oder 2,
Y F oder Cl, und
Q eine Einfachbindung, -CF₂-, -OCF₂- oder -OCHF-
bedeutet,
als weitere Bestandteile 4 bis 30 Komponenten.

5. Medium nach Anspruch 4, dadurch gekennzeichnet daß es neben einem oder mehreren Fluorbenzolderivaten der Formel I'' nach Anspruch 4 als weitere Bestandteile 7 bis 25 Komponenten enthält.

6. Medium nach einem der Ansprüche 4 oder 5, dadurch gekennzeichnet, daß die weiteren Bestandteile ansgewählt sind aus den Verbindungen der Formeln 1, 2, 3, 4 und 5
R'-L-E-R'' 1
R'-L-COO-E-R'' 2
R'-L-OOC-E-R'' 3
R'-L-CH₂CH₂-E-R'' 4
R'-L-C ≡ C-E-R'' 5
worin
L und E, die gleich oder verschieden sein können, jeweils unabhängig voneinander einen bivalenten Rest aus der aus -Phe-,-Cyc-, -Phe-Phe-, Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten, und
R' und R'' jeweils unabhängig voneinander Alkyl, Alkenyl, Alkoxy, Alkoxyalkyl, Alkenyloxy oder Alkanoyloxy mit jeweils 1 bis 8 Kohlenstoffatomen oder R'' auch -F, -Cl, -NCS, -(O)ᵢ-CH_{3-(k + 1)}FₖCl₁ - wobei i 0 oder 1 und (k + 1) 1,2 oder 3 sind - oder -CN bedeuten.

7. Medium nach Anspruch 6, dadurch gekennzeichnet, daß es als weitere Bestandteile mindestens Verbindungen ausgewählt aus zwei der Formeln 1 - 5 enthält.

8. Medium nach Anspruch 6, dadurch gekennzeichnet, daß es einen Bestandteil enthält, worin L Cyc oder Phe und E Phe-Cyc bedeutet.

9. Medium nach Anspruch 8, dadurch gekennzeichnet, daß L Phe bedeutet.

10. Medium nach Anspruch 6, dadurch gekennzeichnet, daß es eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ansgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ansgewählt sind aus der Gruppe -Phe-Cyc-, -Cyc-Cyc-, -G-Phe- und -G-Cyc- enthält.

11. Medium nach einem der Ansprüche 4 bis 10, dadurch gekennzeichnet, daß es mehrere Verbindungen der Formel I'' nach Anspruch 4 enthält.

12. Medium nach Anspruch 11, dadurch gekennzeichnet, daß es drei, vier oder fünf Verbindungen der Formel I'' nach Anspruch 4 enthält.

13. Medium nach Anspruch 6, dadurch gekennzeichnet, daß es (a) 5 - 90 % an einer oder mehreren Verbindungen der Formel I'' nach Anspruch 4, (b) 10 - 80 % an Verbindungen ausgewählt aus der Gruppe der Formeln 1 bis 5, worin R'' -F, -Cl, -NCS oder -(O)ᵢ-CH₃₋₍ₖ₊₁₎FₖCl₁ bedeutet (Gruppe B) und (c) 0-30 % an Verbindungen ansgewählt aus der Gruppe der Formeln 1 bis 5, worin R' und R'' jeweils unabhängig voneinander Alkyl, Alkenyl, Alkoxy, Alkoxyalkyl, Alkenyloxy oder Alkanoyloxy mit 1 bis 8 Kohlenstoffatomen bedeutet (Gruppe A), enthält.

14. Medium nach Anspruch 13, dadurch gekennzeichnet, daß es Verbindungen ausgewählt aus der Gruppe der Formeln 1 bis 5 enthält, worin einer der Reste L und E durch Fluor substituiertes 1,4-Phenylen enthält.

15. Medium nach Anspruch 13, dadurch gekennzeichnet, daß es aus Verbindungen der Formel I'' nach Anspruch 4 und Verbindungen der Gruppe B besteht.

16. Medium nach einem der Ansprüche 4 bis 15, mit der Maßgabe, daß ein solches bestehend aus Verbindungen der Formel I'' und Verbindungen der Formel 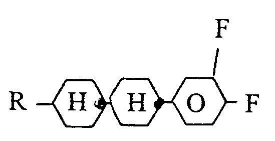 worin R geradkettiges Alkyl bedeutet, ausgenommen sind.

17. Medien nach einen der Ansprüche 4 bis 16, dadurch gekennzeichnet, daß es mehr als 40 % an Verbindungen der Formel I'' nach Anspruch 4 enthält.

18. Medium nach Anspruch 17, dadurch gekennzeichnet, daß der Anteil der Verbindungen der Formel I'' nach Anspruch 4 45 bis 90 % ist.

19. Flüssigkristall-Anzeigeelement, dadurch gekennzeichnet, daß es flüssigkristallines Medium nach einem der Ansprüche 3 bis 18 enthält.

20. Elektrooptisches Anzeigeelement, dadurch gekennzeichnet, daß es als Dielektrikum ein flüssigkristallines Medium nach einem der Ansprüche 3 bis 18 enthält.

## Patentansprüche (Patentansprüche für folgende(n) Vertragsstaat(en): DK, ES)

1. Fluorbenzolderivate der Formel I, 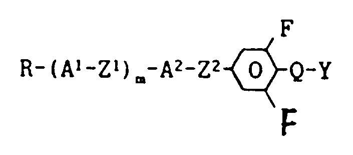 worin
R H, einen unsubstituierten, einen einfach durch CN oder CF₃ oder einen mindestens einfach durch Halogen substituierten Alkyl- oder Alkenylrest mit, 1 bis 15 C-Atomen, wobei in diesen Resten auch eine oder mehrere CH₂-Gruppen jeweils unabhängig von einander durch -O-, -S-, 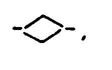 -CO-, -CO-O-, -O-CO- oder -O-CO-O- so ersetzt sein können, daß O-Atome nicht direkt miteinander verknüpft sind,
A¹ und A² jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Fluor substituiert sein können,
Z¹ und Z² jeweils unabhängig voneinander -CO-O-, -O-CO-, -CH₂O-, -OCH₂-, -CH₂CH₂-, -CH=CH-, -C≡C- oder eine Einfachbindung, einer der Reste Z¹ und Z² auch -(CH₂)₄- oder -CH=CH-CH₂CH₂-,
m 0, 1 oder 2,
Y F oder Cl, und
Q eine Einfachbindung, -CF₂-, -OCF₂- oder -OCHF-
bedeutet.

2. Verwendung von Verbindungen der Formel I als Komponenten flüssigkristalliner Medien.

3. Flüssigkristallines Medium mit mindestens zwei flüssigkristallinen Komponenten, dadurch gekennzeichnet, daß sie mindestens eine Verbindung der Formel I enthält.

4. Flüssigkristallines Medium enthaltend neben einem oder mehreren Fluorbenzolderivaten der Formel I'' 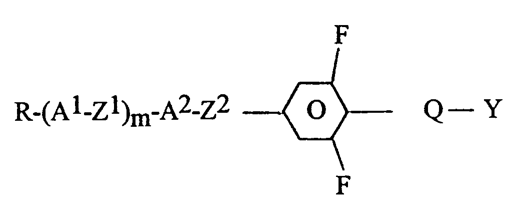 worin
R H, einen unsubstituierten, einen einfach durch CN oder CF₃ oder einen mindestens einfach durch Halogen substituierten Alkyl- oder Alkenylrest mit 1 bis 15 C-Atomen wobei in diesen Resten auch eine oder mehrere CH₂-Gruppen jeweils unabhängig voneinander durch -O-, -S-, 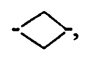 -CO-, -CO-O-, -O-CO- oder -O-CO-O- so ersetzt sein können, daß O-Atome nicht direkt miteinander verknüpft sind,
A¹ und A² jeweils unabhängig voneinander einen
(a) trans-1,4-Cyclohexylenrest, worin auch eine oder mehrere nicht benachbarte CH₂-Gruppen durch-O- und/oder -S-ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo (2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Rest (a) und (b) durch CN oder Fluor substituiert sein können,
Z¹ und Z² jeweils unabhängig voneinander -CO-O-, -O-CO-, -CH₂O-, -OCH₂-, -CH₂CH₂-, -CH=CH.-, -C≡C-oder eine Einfachbindung, einer der Reste Z1 und Z2 auch -(CH₂)₄- oder -CH=CH-CH₂CH₂-,
m 0, 1 oder 2,
Y F oder Cl, und
Q eine Einfachbindung, -CF₂-, -OCF₂- oder -OCHF-
bedeutet,
als weitere Bestandteile 4 bis 30 Komponenten.

5. Medium nach Anspruch 4, dadurch gekennzeichnet, daß es neben einem oder mehreren Fluorbenzolderivaten der Formel I'' nach Anspruch 4 als weitere Bestandteile 7 bis 25 Komponenten enthält.

6. Medium nach einem der Ansprüche 4 oder 5, dadurch gekennzeichnet, daß die weiteren Bestandteile ausgewählt sind aus den Verbindungen der Formeln 1, 2, 3, 4 und 5
R'-L-E-R'' 1
R'-L-COO-E-R'' 2
R'-L-OOC-E-R'' 3
R'-L-CH₂CH₂-E-R'' 4
R'-L-C ≡ C-E-R'' 5
worin
L und E, die gleich oder verschieden sein können, jeweils unabhängig voneinander einen bivalenten Rest aus der aus -Phe-,-Cyc-, -Phe-Phe-, Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten, und
R' und R'' jeweils unabhängig voneinander Alkyl, Alkenyl, Alkoxy, Alkoxyalkyl, Alkenyloxy oder Alkanoyloxy mit jeweils 1 bis 8 Kohlenstoffatomen oder R'' auch -F, -Cl, -NCS, -(O)ᵢ-CH₃₋₍ₖ₊₁₎FₖCl₁ - wobei i 0 oder 1 und (k + 1) 1,2 oder 3 sind - oder -CN bedeuten.

7. Medium nach Anspruch 6, dadurch gekennzeichnet, daß es als weitere Bestandteile mindestens Verbindungen ausgewählt aus zwei der Formeln 1 - 5 enthält.

8. Medium nach Anspruch 6, dadurch gekennzeichnet, daß es einen Bestandteil enthält, worin L Cyc oder Phe und E Phe-Cyc bedeutet.

9. Medium nach Anspruch 8, dadurch gekennzeichnet, daß L Phe bedeutet.

10. Medium nach Anspruch 6, dadurch gekennzeichnet, daß es eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc- enthält.

11. Medium nach einem der Ansprüche 4 bis 10, dadurch gekennzeichnet, daß es mehrere Verbindungen der Formel I'' nach Anspruch 4 enthält.

12. Medium nach Anspruch 11, dadurch gekennzeichnet, daß es drei, vier oder fünf Verbindungen der Formel I'' nach Anspruch 4 enthält.

13. Medium nach Anspruch 6, dadurch gekennzeichnet, daß es (a) 5-90% an einer oder mehreren Verbindungen der Formel I'' nach Anspruch 4, (b) 10 - 80% an Verbindungen ausgewählt aus der Gruppe der Formeln 1 bis 5, worin R''-F, -Cl, -NCS oder -(O)ᵢ-CH_{3-(k + 1)}FₖCl₁ bedeutet (Gruppe B) und (c) 0-30 % an Verbindungen ausgewählt aus der Gruppe der Formeln 1 bis 5, worin R' und R'' jeweils unabhängig voneinander Alkyl, Alkenyl, Alkoxy, Alkoxyalkyl, Alkenyloxy oder Alkanoyloxy mit 1 bis 8 Kohlenstoffatomen bedeutet (Gruppe A), enthält.

14. Medium nach Anspruch 13, dadurch gekennzeichnet, daß es Verbindungen ausgewählt aus der Gruppe der Formeln 1 bis 5 enthält, worin einer der Reste L und E durch Fluor substituiertes 1,4-Phenylen enthält.

15. Medium nach Anspruch 13, dadurch gekennzeichnet, daß es aus Verbindungen der Formel I'' nach Anspruch 4 und Verbindungen der Gruppe B besteht.

16. Medium nach einem der Ansprüche 4 bis 15, mit der Maßgabe, daß ein solches bestehend aus Verbindungen der Formel I'' und Verbindungen der Formel 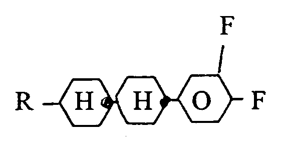 worin R geradkettiges Alkyl bedeutet, ausgenommen sind.

17. Medien nach einen der Ansprüche 4 bis 16, dadurch gekennzeichnet, daß es mehr als 40 % an Verbindungen der Formel I'' nach Anspruch 4 enthält.

18. Medium nach Anspruch 17, dadurch gekennzeichnet daß der Anteil der Verbindungen der Formel I'' nach Anspruch 4 45 bis 90 % ist.

19. Flüssigkristall-Anzeigeelement, dadurch gekennzeichnet, daß es flüssigkristallines Medium nach einem der Ansprüche 3 bis 18 enthält.

20. Elektrooptisches Anzeigeelement, dadurch gekennzeichnet, daß es als Dielektrikum ein flüssigkristallines Medium nach einem der Ansprüche 3 bis 18 enthält.

## Claims (Claims for the following Contracting State(s): AT, BE, CH, LI, DE, FR, GB, IT, LU, NL, SE)

1. Fluorobenzene derivatives of the formula I, 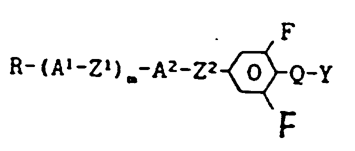 in which
R is H, an alkyl or alkenyl radical of 1 to 15 carbon atoms which is unsubstituted or monosubstituted by CN or CF₃ or at least monosubstituted by halogen, it being possible for one or more CH₂ groups in these radicals also to be replaced, in each case independently of one another, by -O-, -S-, 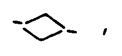 -CO-, -CO-O-, -O-CO- or -O-CO-O- in such a manner that O atoms are not linked directly to one another,
A¹ and A², in each case independently of one another, are a
(a) trans-1,4-cyclohexylene radical, in which one or more non-adjacent CH₂ groups can also be replaced by -O-and/or -S-,
(b) 1,4-phenylene radical, in which one or two CH groups can also be replaced by N,
(c) radical from the group comprising 1,4-cyclohexenylene, 1,4-bicyclo(2.2.2)octylene, piperidine-1,4-diyl, naphthalene-2,6-diyl, decahydronaphthalene-2,6-diyl and 1,2,3,4-tetrahydronaphthalene-2,6-diyl,
it being possible for the radicals (a) and (b) to be substituted by CN or fluorine,
Z¹ and Z², in each case independently of one another, are -CO-O-, -O-CO-, -CH₂O-, -OCH₂-, -CH₂CH₂-, -CH=CH-, -C≡C- or a single bond, one of the radicals Z¹ and Z² is also -(CH₂)₄- or -CH=CH-CH₂CH₂-,
m is 0, 1 or 2,
Y is F or Cl, and
Q is a single bond, -CF₂-, -OCF₂- or -OCHF-,
with the proviso that the following compounds are excluded:
(a) 4-propyl-4'-(3,5-difluoro-4-difluoromethoxyphenyl)-trans, trans-bicyclohexyl
(b) 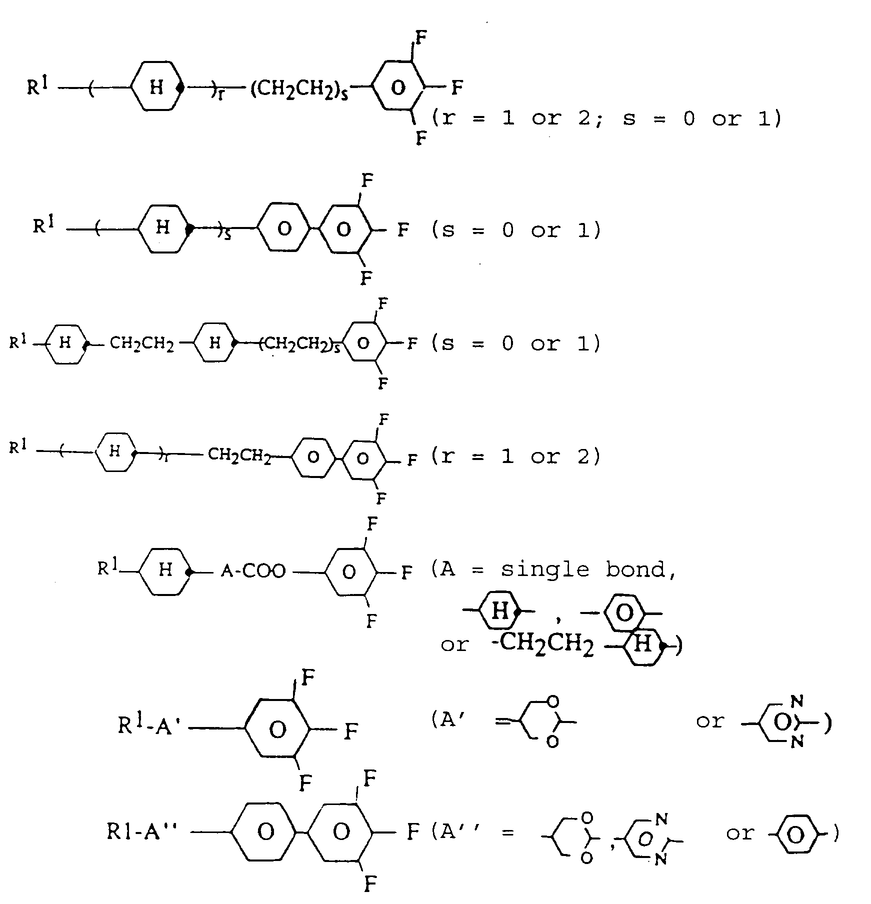
where R1 in each case is an alkyl group having 1 to 10 C atoms.

2. Use of compounds of the formula I as components of liquid-crystalline media.

3. Liquid-crystalline medium containing at least two liquid-crystalline components, characterized in that it contains at least one compound of the formula I.

4. Liquid-crystalline medium containing, in addition to one or more fluorobenzene derivatives of the formula I'' 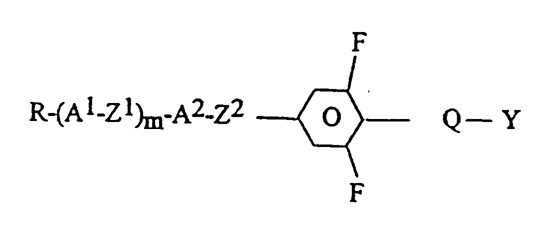 wherein
R is H, an alkyl or alkenyl radical of 1 to 15 carbon atoms which is unsubstituted or monosubstituted by CN or CF₃ or at least monosubstituted by halogen, it being possible for one or more CH₂ groups in these radicals also to be replaced, in each case independently of one another, by -O-, -S-, 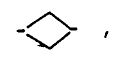 -CO-, -CO-O-, -O-CO- or -O-CO-O- in such a manner that O atoms are not linked directly to one another,
A¹ and A², in each case independently of one another, are a
(a) trans-1,4-cyclohexylene radical, in which one or more non-adjacent CH₂ groups can also be replaced by -O-and/or -S-,
(b) 1,4-phenylene radical, in which one or two CH groups can also be replaced by N,
(c) radical from the group comprising 1,4-cyclohexenylene, 1,4-bicyclo(2.2.2)octylene, piperidine-1,4-diyl, naphthalene-2,6-diyl, decahydronaphthalene-2,6-diyl and 1,2,3,4-tetrahydronaphthalene-2,6-diyl,
it being possible for the radicals (a) and (b) to be substituted by CN or fluorine,
Z¹ and Z², in each case independently of one another, are -CO-O-, -O-CO-, -CH₂O-, -OCH₂-, -CH₂CH₂-, -CH=CH-, -C≡C- or a single bond, one of the radicals Z1 and Z2 is also -(CH₂)₄- or -CH=CH-CH₂CH₂-,
m is 0, 1 or 2,
Y is F or Cl, and
Q is a single bond, -CF₂-, -OCF₂- or -OCHF-,
4 to 30 components as further constituents.

5. Medium according to Claim 4, characterized in that, in addition to one or more fluorobenzene derivatives of the formula I'' according to Claim 4, it contains 7 to 25 components as further constituents.

6. Medium according to one of Claims 4 or 5, characterized in that the further constituents are selected from the compounds of the formulae 1, 2, 3, 4 and 5
R'-L-E-R'' 1
R'-L-COO-E-R'' 2
R'-L-OOC-E-R'' 3
R'-L-CH₂CH₂-E-R'' 4
R'-L-C≡C-E-R'' 5
in which
L and E, which can be identical or different, in each case independently of one another, are a divalent radical from the group formed by -Phe-, -Cyc-, -Phe-Phe-, -Phe-Cyc-, -Cyc-Cyc-, -Pyr-, -Dio-, -G-Phe- and -G-Cyc- and their mirror images, in which Phe is unsubstituted or fluorine-substituted 1,4-phenylene, Cyc is trans-1,4-cyclohexylene or 1,4-cyclohexenylene, Pyr is pyrimidine-2,5-diyl or pyridine-2,5-diyl, Dio is 1,3-dioxane-2,5-diyl and G is 2-(trans-1,4-cyclohexyl)ethyl, pyrimidine-2,5-diyl, pyridine-2,5-diyl or 1,3-dioxane-2,5-diyl, and
R' and R'' are in each case independently of one another alkyl, alkenyl, alkoxy, alkoxyalkyl, alkenyloxy or alkanoyloxy in each case having 1 to 8 carbon atoms or R'' is -F, -Cl, -NCS or -(O)ᵢ-CH₃₋₍ₖ₊₁₎FₖCl₁, where i is 0 or 1 and (k+1) is 1, 2 or 3, or is -CN.

7. Medium according to Claim 6, characterized in that, as further constituents, it contains at least compounds selected from two of the formulae 1 - 5.

8. Medium according to Claim 6, characterized in that it contains a constituent in which L is Cyc or Phe and E is Phe-Cyc.

9. Medium according to Claim 8, characterized in that L is Phe.

10. Medium according to Claim 6, characterized in that it contains one or more components selected from the compounds of the formulae 1, 2, 3, 4 and 5, in which L and E are selected from the group consisting of Cyc, Phe and Pyr and simultaneously one or more components selected from the compounds of the formulae 1, 2, 3, 4 and 5, in which one of the radicals L and E is selected from the group Cyc, Phe and Pyr and the other radical is selected from the group -Phe-Phe-, -Phe-Cyc-, Cyc-Cyc-, -G-Phe- and G-Cyc-, and optionally one or more components selected from the compounds of the formulae 1, 2, 3, 4 and 5, in which the radicals L and E are selected from the group -Phe-Cyc-, -Cyc-Cyc-, -G-Phe- and G-Cyc-.

11. Medium according to one of Claims 4 to 10, characterized in that it contains several compounds of the formula I'' according to Claim 4.

12. Medium according to Claim 11, characterized in that it contains three, four or five compounds of the formula I'' according to Claim 4.

13. Medium according to Claim 6, characterized in that it contains (a) 5 - 90 % of one or more compounds of the formula I'' according to Claim 4, (b) 10 - 80 % of compounds selected from the group of the formulae 1 to 5, in which R'' is -F, -Cl, -NCS or -(O)ᵢ-CH₃₋₍ₖ₊₁₎FₖCl₁ (group B) and (c) 0-30 % of compounds selected from the group of the formulae 1 to 5, in which R' and R'' in each case independently of one another are alkyl, alkenyl, alkoxy, alkoxyalkyl, alkenyloxy or alkanoyloxy having 1 to 8 carbon atoms (group A).

14. Medium according to Claim 13, characterized in that it contains compounds selected from the group of the formulae 1 to 5, in which one of the radicals L and E contains 1,4-phenylene substituted by fluorine.

15. Medium according to Claim 13, characterized in that it consists of compounds of the formula I'' according to Claim 4 and compounds of the group B.

16. Medium according to one of Claims 4 to 15, with the proviso that one consisting of compounds of the formula I'' and compounds of the formula 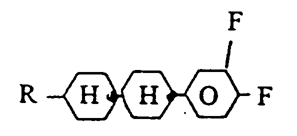 in which R is straight-chain alkyl, are [sic] excluded.

17. Media according to one of Claims 4 to 16, characterized in that it contains more than 40 % of compounds of the formula I'' according to Claim 4.

18. Medium according to Claim 17, characterized in that the proportion of the compounds of the formula I'' according to Claim 4 is 45 to 90 %.

19. Liquid-crystal display element, characterized in that it contains a liquid-crystalline medium according to one of Claims 3 to 18.

20. Electrooptical display element, characterized in that it contains, as dielectric, a liquid-crystalline medium according to one of Claims 3 to 18.

## Claims (Claims for the following Contracting State(s): DK, ES)

1. Fluorobenzene derivatives of the formula I, 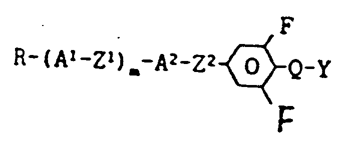 in which
R is H, an alkyl or alkenyl radical of 1 to 15 carbon atoms which is unsubstituted or monosubstituted by CN or CF₃ or at least monosubstituted by halogen, it being possible for one or more CH₂ groups in these radicals also to be replaced, in each case independently of one another, by -O-, -S-, 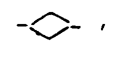 -CO-, -CO-O-, -O-CO- or -O-CO-O- in such a manner that O atoms are not linked directly to one another,
A¹ and A², in each case independently of one another, are a
(a) trans-1,4-cyclohexylene radical, in which one or more non-adjacent CH₂ groups can also be replaced by -O-and/or -S-,
(b) 1,4-phenylene radical, in which one or two CH groups can also be replaced by N,
(c) radical from the group comprising 1,4-cyclohexenylene, 1,4-bicyclo[2.2.2]-octylene, piperidine-1,4-diyl, naphthalene-2,6-diyl, decahydronaphthalene-2,6-diyl and 1,2,3,4-tetrahydronaphthalene-2,6-diyl,
it being possible for the radicals (a) and (b) to be substituted by CN or fluorine,
Z¹ and Z², in each case independently of one another, are -CO-O-, -O-CO-, -CH₂O-, -OCH₂-, -CH₂CH₂-, -CH=CH-, -C≡C- or a single bond, one of the radicals Z1 and Z2 is also -(CH₂)₄- or -CH=CH-CH₂CH₂-,
m is 0, 1 or 2,
Y is F or Cl, and
Q is a single bond, -CF₂-, -OCF₂- or -OCHF-.

2. Use of compounds of the formula I as components of liquid-crystalline media.

3. Liquid-crystalline medium containing at least two liquid-crystalline components, characterized in that it contains at least one compound of the formula I.

4. Liquid-crystalline medium containing, in addition to one or more fluorobenzene derivatives of the formula I'' 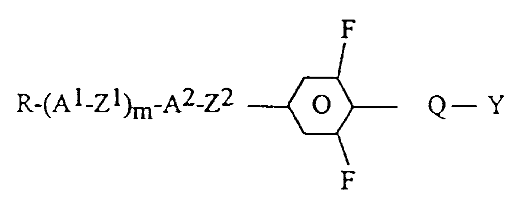 wherein
R is H, an alkyl or alkenyl radical of 1 to 15 carbon atoms which is unsubstituted or monosubstituted by CN or CF₃ or at least monosubstituted by halogen, it being possible for one or more CH₂ groups in these radicals also to be replaced, in each case independently of one another, by -O-, -S-, 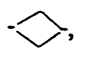 -CO-, -CO-O-, -O-CO- or -O-CO-O- in such a manner that O atoms are not linked directly to one another,
A¹ and A², in each case independently of one another, are a
(a) trans-1,4-cyclohexylene radical, in which one or more non-adjacent CH₂ groups can also be replaced by -O-and/or -S-,
(b) 1,4-phenylene radical, in which one or two CH groups can also be replaced by N,
(c) radical from the group comprising 1,4-cyclohexenylene, 1,4-bicyclo(2.2.2)octylene, piperidine-1,4-diyl, naphthalene-2,6-diyl, decahydronaphthalene-2,6-diyl and 1,2,3,4-tetrahydronaphthalene-2,6-diyl,
it being possible for the radicals (a) and (b) to be substituted by CN or fluorine,
Z¹ and Z², in each case independently of one another, are -CO-O-, -O-CO-, -CH₂O-, -OCH₂-, -CH₂CH₂-, -CH=CH-, -C≡C- or a single bond, one of the radicals Z1 and Z2 is also -(CH₂)₄- or -CH=CH-CH₂CH₂-,
m is 0, 1 or 2,
Y is F or Cl, and
Q is a single bond, -CF₂-, -OCF₂- or -OCHF-,
4 to 30 components as further constituents.

5. Medium according to Claim 4, characterized in that, in addition to one or more fluorobenzene derivatives of the formula I'' according to Claim 4, it contains 7 to 25 components as further constituents.

6. Medium according to one of Claims 4 or 5, characterized in that the further constituents are selected from the compounds of the formulae 1, 2, 3, 4 and 5
R'-L-E-R'' 1
R'-L-COO-E-R'' 2
R'-L-OOC-E-R'' 3
R'-L-CH₂CH₂-E-R'' 4
R'-L-C≡C-E-R'' 5
in which
L and E, which can be identical or different, in each case independently of one another, are a divalent radical from the group formed by -Phe-, -Cyc-, -Phe-Phe-, -Phe-Cyc-, -Cyc-Cyc-, -Pyr-, -Dio-, -G-Phe- and -G-Cyc- and their mirror images, in which Phe is unsubstituted or fluorine-substituted 1,4-phenylene, Cyc is trans-1,4-cyclohexylene or 1,4-cyclohexenylene, Pyr is pyrimidine-2,5-diyl or pyridine-2,5-diyl, Dio is 1,3-dioxane-2,5-diyl and G is 2-(trans-1,4-cyclohexyl)ethyl, pyrimidine-2,5-diyl, pyridine-2,5-diyl or 1,3-dioxane-2,5-diyl, and
R' and R'' are in each case independently of one another alkyl, alkenyl, alkoxy, alkoxyalkyl, alkenyloxy or alkanoyloxy in each case having 1 to 8 carbon atoms or R'' is -F, -Cl, -NCS or -(O)ᵢ-CH₃₋₍ₖ₊₁₎FₖCl₁, where i is 0 or 1 and (k+1) is 1, 2 or 3, or is -CN.

7. Medium according to Claim 6, characterized in that, as further constituents, it contains at least compounds selected from two of the formulae 1 - 5.

8. Medium according to Claim 6, characterized in that it contains a constituent in which L is Cyc or Phe and E is Phe-Cyc.

9. Medium according to Claim 8, characterized in that L is Phe.

10. Medium according to Claim 6, characterized in that it contains one or more components selected from the compounds of the formulae 1, 2, 3, 4 and 5, in which L and E are selected from the group consisting of Cyc, Phe and Pyr and simultaneously one or more components selected from the compounds of the formulae 1, 2, 3, 4 and 5, in which one of the radicals L and E is selected from the group Cyc, Phe and Pyr and the other radical is selected from the group -Phe-Phe-, -Phe-Cyc-, Cyc-Cyc-, -G-Phe- and G-Cyc-, and optionally one or more components selected from the compounds of the formulae 1, 2, 3, 4 and 5, in which the radicals L and E are selected from the group -Phe-Cyc-, -Cyc-Cyc-, -G-Phe- and G-Cyc-.

11. Medium according to one of Claims 4 to 10, characterized in that it contains several compounds of the formula I'' according to Claim 4.

12. Medium according to Claim 11, characterized in that it contains three, four or five compounds of the formula I'' according to Claim 4.

13. Medium according to Claim 6, characterized in that it contains (a) 5 - 90 % of one or more compounds of the formula I'' according to Claim 4, (b) 10 - 80 % of compounds selected from the group of the formulae 1 to 5, in which R'' is -F, -Cl, -NCS or -(O)ᵢ-CH₃₋₍ₖ₊₁₎FₖCl₁ (group B) and (c) 0-30 % of compounds selected from the group of the formulae 1 to 5, in which R' and R'' in each case independently of one another are alkyl, alkenyl, alkoxy, alkoxyalkyl, alkenyloxy or alkanoyloxy having 1 to 8 carbon atoms (group A).

14. Medium according to Claim 13, characterized in that it contains compounds selected from the group of the formulae 1 to 5, in which one of the radicals L and E contains 1,4-phenylene substituted by fluorine.

15. Medium according to Claim 13, characterized in that it consists of compounds of the formula I'' according to Claim 4 and compounds of the group B.

16. Medium according to one of Claims 4 to 15, with the proviso that one consisting of compounds of the formula I'' and compounds of the formula 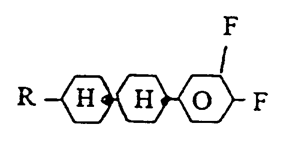 in which R is straight-chain alkyl, are [sic] excluded.

17. Media according to one of Claims 4 to 16, characterized in that it contains more than 40 % of compounds of the formula I'' according to Claim 4.

18. Medium according to Claim 17, characterized in that the proportion of the compounds of the formula I'' according to Claim 4 is 45 to 90 %.

19. Liquid-crystal display element, characterized in that it contains a liquid-crystalline medium according to one of Claims 3 to 18.

20. Electrooptical display element, characterized in that it contains, as dielectric, a liquid-crystalline medium according to one of Claims 3 to 18.

## Revendications (Revendications pour l'(les) Etat(s) contractant(s) suivant(s): AT, BE, CH, LI, DE, FR, GB, IT, LU, NL, SE)

1. Dérivés du fluorobenzène répondant à la formule I 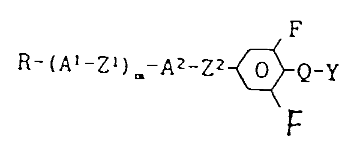 dans laquelle
R représente H, un radical alkyle ou alcènyle en C1-C15 non substitué, mono-substitué par CN ou CF₃ ou au moins mono-substitué par des halogènes, et dans lequel également un ou plusieurs groupes CH₂ peuvent être remplacés chacun, indépendemment les uns des autres, par -O-, -S-, -CO-, -CO-O-, -O-CO- ou -O-CO-O- sous réserve cependant que des atomes d'oxygène ne peuvent pas être reliés directement entre eux, A¹ et A² représentent chacun, indépendamment l'un de l'autre,
(a) un groupe trans-1,4-cyclohexylène dans lequel également un ou plusieurs CH₂ non voisins peuvent être remplacés par -O- et/ou -S-,
(b) un groupe 1,4-phénylène dans lequel également un ou deux groupes CH peuvent être remplacés par N,
(c) un groupe choisi parmi les groupes 1,4-cyclohexenylène, 1,4-bicyclo(2,2,2)octylène, pipéridine-1,4-diyle, naphtalène-2,6-diyle, décahydronaphtalène-2,6-diyle et 1,2,3,4-tétrahydronaphtalène-2,6-diyle, les groupes (a) et (b) pouvant être substitués par CN ou le fluor, Z¹ et Z² représentent chacun, indépendamment l'un de l'autre -CO-O-, -O-CO-, -CH₂O-, -OCH₂-, -CH₂CH₂-, -CH=CH-, -C≡C- ou une liaison simple, l'un des symboles Z¹ et Z² pouvant également représenter -(CH₂)₄- ou -CH=CH-CH₂CH₂-,
m est égal à 0, 1 ou 2,
Y représente F ou Cl et
Q représente une liaison simple, -CF₂, -OCF₂- ou -OCHF-,
sous réserve que les composés suivants sont exclus :
(a) le 4-propyl-4'-(3,5-difluoro-4-difluorométhoxyphényl)-trans, transbicyclohexyle, 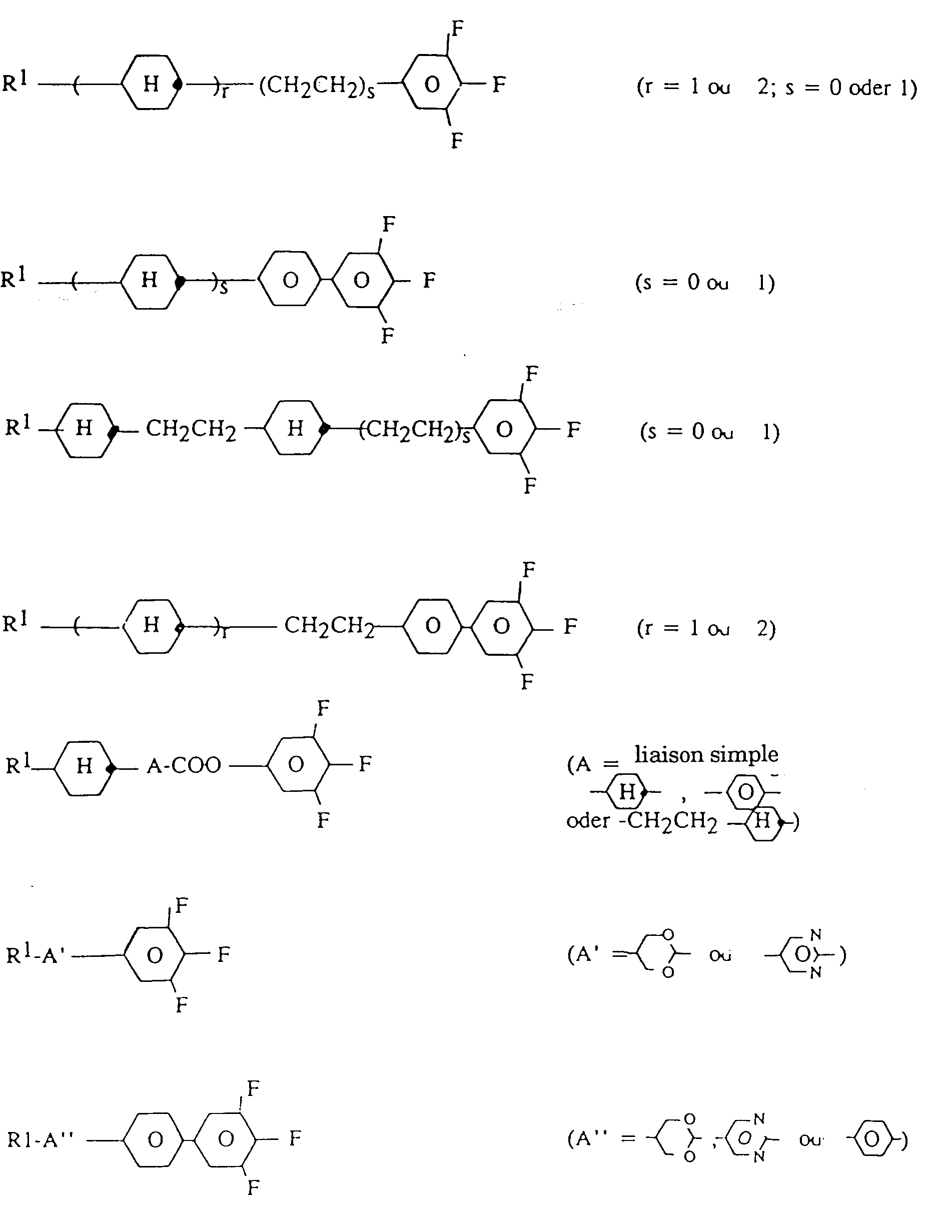
R représentant clans chaque cas un groupe alkyle en C1-C10.

2. Utilisation des composés de formule I en tant que composants de milieux à cristaux liquides.

3. Milieu à cristaux liquides à au moins deux composants à cristaux liquides, caractérisé en ce qu'il contient au moins un composé de formule I.

4. Milieu à cristaux liquides contenant, en plus d'un ou plusieurs dérivés du fluorobenzène répondant à la formule I'' 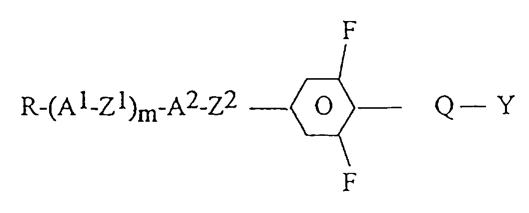 dans laquelle
R représente H, un radical alkyle ou alcènyle en C1-C15 non substitué, mono-substitué par CN ou CF₃ ou au moins mono-substitué par des halogènes, et dans lequel également un ou plusieurs groupes CH₂ peuvent être remplacés chacun, indépendamment les uns des autres, par -O-, -S-, 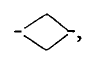 -CO-, -CO-O-, O-CO- ou -O-CO-O-sous réserve toutefois que des atomes d'oxygène ne peuvent pas être reliés directement entre eux,
A¹ et A² représentent chacun, indépendamment l'un de l'autre,
(a) un groupe trans-1,4-cyclohexylène dans lequel également un ou plusieurs groupes CH₂ non voisins peuvent être remplacés par -O- et/ou -S-,
(b) un groupe 1,4-phénylène dans lequel également un ou deux groupes CH peuvent être remplacés par N,
(c) un groupe choisi parmi les groupes 1,4-cyclohexenylène, 1,4-bicyclo(2,2,2)octylène, pipéridine-1,4-diyle, naphtalène-2,6-diyle, décahydronaphtalène-2,6-diyle et 1,2,3,4-tétrahydronaphtalène-2,6-diyle, les groupes (a) et (b) pouvant être substitués par CN ou le fluor,
Z¹ et Z² représentent chacun, indépendamment l'un de l'autre -CO-O-, -O-CO-, -CH₂O-, -OCH₂, -CH₂CH₂-, -CH=CH.-, -C≡C-ou une liaison simple, l'un des symboles Z¹ et Z² pouvant également représenter -(CH₂)₄- ou -CH=CH-CH₂CH₂-,
m est égal à 0, 1 ou 2,
Y représente F ou Cl et
Q représente une liaison simple, -CF₂-, -OCF₂- ou -OCHF-,
de 4 à 30 autres composants.

5. Milieu selon revendication 4, caractérisé en ce que, en plus d'un ou plusieurs dérivés du fluorobenzène de formule I'' selon la revendication 4, il contient de 7 à 25 autres composants.

6. Milieu selon une des revendications 4 ou 5, caractérisé en ce que ces autres composants sont choisis parmi les composés répondant aux formules 1, 2, 3, 4 et 5
R'-L-E-R'' 1
R'-L-COO-E-R'' 2
R'-L-OOC-E-R'' 3
R'-L-CH₂CH₂-E-R'' 4
R'-L-C ≡ C-E-R'' 5
dans lesquelles
L et E, qui peuvent avoir des significations identiques ou différentes, représentent chacun, indépendamment l'un de l'autre, un groupe bivalent choisi parmi -Phe-, -Cyc-, -Phe-Phe-, Phe-Cyc-, -Cyc-Cyc-, -Pyr-, -Dio-, -G-Phe- et -G-Cyc- et leurs images spéculaires, Phe représentant un groupe 1,4-phénylène non substitué ou substitué par le fluor, Cyc un groupe trans-1,4-cyclohexylène ou 1,4-cyclohexénylène, Pyr un groupe pyrimidine-2,5-diyle ou pyridine-2,5-diyle, Dio un groupe 1,3-dioxanne-2,5-diyle et G un groupe 2-(trans-1,4-cyclohexyl)-éthyle, pyrimidine-2,5-diyle, pyridine-2,5-diyle ou 1,3-dioxanne-2,5-diyle et
R' et R'' représentent chacun, indépendamment l'un de l'autre, un groupe alkyle, alcènyle, alcoxy, alcoxyalkyle, alcènyloxy ou alcanoyloxy contenant chacun 1 à 8 atomes de carbone,
R'' pouvant également représenter -F, -Cl, -NCS,
-(O)ᵢ-CH₃₋₍ₖ₊₁₎FₖCl₁ -dans lequel i est égal à 0 ou 1 et (k + 1) est égal à 1,2 ou 3 - ou -CN.

7. Milieu selon revendication 6, caractérisé en ce qu'il contient en tant qu'autres composants au moins deux composés choisis parmi ceux qui répondent aux formules 1 à 5.

8. Milieu selon revendication 6, caractérisé en ce qu'il contient un composant pour lequel L représente Cyc ou Phe et E représente Phe-Cyc.

9. Milieu selon revendication 8, caractérisé en ce que L représente Phe.

10. Milieu selon revendication 6, caractérisé en ce qu'il contient un ou plusieurs composants choisis parmi les composés répondant aux formules 1, 2, 3, 4 et 5 dans lesquelles L et E sont choisis parmi Cyc, Phe et Pyr et, simultanément, un ou plusieurs composants choisis parmi les composés répondant aux formules 1, 2, 3, 4 et 5 dans lesquelles l'un des symboles L et E représente un groupe choisi parmi Cyc, Phe et Pyr et l'autre un composant choisi parmi -Phe-Phe-, -Phe-Cyc-, Gyc-Cyc-, -G-Phe- et -G-Cyc, et le cas échéant un ou plusieurs composants choisis parmi les composés répondant aux formules 1, 2, 3, 4 et 5 dans lesquelles les symboles L et E représentent des groupes choisis parmi -Phe-Cyc-, -Cyc-Cyc-, -G-Phe- et-G-Cyc-.

11. Milieu selon une des revendications 4 à 10, caractérisé en ce qu'il contient plusieurs composés de formule I'' selon revendication 4.

12. Milieu selon revendication 11, caractérisé en ce qu'il contient trois, quatre ou cinq composés de formule I'' selon revendication 4.

13. Milieu selon revendication 6, caractérisé en ce qu'il contient (a) 5 à 90 % d'un ou plusieurs composés de formule I'' selon revendication 4, (b) 10 à 80 % de composés choisis parmi ceux qui répondent aux formules 1 à 5 dans lesquelles R'' représente -F, -Cl, -NCS ou -(O)i-CH₃₋₍ₖ₊₁₎FₖCl₁ (groupe B) et (c) 0 à 30 % de composés choisis parmi ceux qui répondent aux formules 1 à 5 dans lesquelles R' et R'' représentent chacun, indépendamment l'un de l'autre, un radical alkyle, alcènyle, alcoxy, alcoxyalkyle, alcényloxy ou alcanoyloxy en C1-C8 (groupe A).

14. Milieu selon revendication 13, caractérisé en ce qu'il contient des composés choisis parmi ceux qui répondent aux formules 1 à 5 dans lesquelles l'un des symboles L et E représente un groupe 1,4-phénylène substitué par le fluor.

15. Milieu selon revendication 13, caractérisé en ce qu'il consiste en composés de formule I'' selon revendication 4 et composés du groupe B.

16. Milieu selon une des revendications 4 à 15, sous réserve qu'un tel milieu, consistant en composés de formule I'' et composés de formule 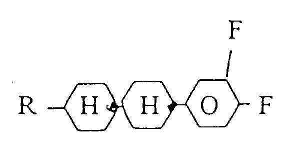 dans laquelle R représente un groupe alkyle à chaîne droite, est exclu.

17. Milieux selon une des revendications 4 à 16, caractérisés en ce qu'ils contiennent plus de 40 % de composés de formule I'' selon revendication 4.

18. Milieu selon revendication 17, caractérisé en ce que la proportion des composés de formule I'' selon revendication 4 est de 45 à 90 %.

19. Elément d'affichage à cristaux liquides caractérisé en ce qu'il contient un milieu à cristaux liquides selon une des revendications 3 à 18.

20. Elément d'affichage électro-optique caractérisé en ce qu'il contient en tant que diélectrique un milieu à cristaux liquides selon une des revendications 3 à 18.

## Revendications (Revendications pour l'(les) Etat(s) contractant(s) suivant(s): DK, ES)

1. Dérivés clu fluorobenzène répondant à la formule I 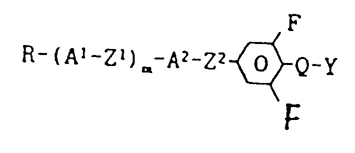 dans laquelle
R représente H, un radical alkyle ou alcènyle en C1-C15 non substitué, mono-substitué par CN ou CF₃ ou au moins mono-substitué par des halogènes, et dans lequel un ou plusieurs groupes CH₂ peuvent être remplacés chacun, indépendamment les uns des autres, par -O-, -S-, 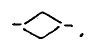 -CO-, -CO-O-, -O-CO- ou -O-CO-O-sous réserve que des atomes d'oxygène ne peuvent pas être reliés directement entre eux,
A¹ et A² représentent chacun, indépendamment l'un de l'autre,
(a) un groupe trans-1,4-cyclohexylène dans lequel également un ou plusieurs groupes CH₂ non voisins peuvent être remplacés par -O- et/ou -S-,
(b) un groupe 1,4-phénylène dans lequel également un ou deux groupes CH peuvent être remplacés par N,
(c) un groupe choisi parmi les groupes 1,4-cyclohexénylène, 1,4-bicyclo(2,2,2)-octylène, pipéridine-1,4-diyle, naphtalène-2,6-diyle, décahydronaphtalène-2,6-diyle et 1,2,3,4-tétrahydronaphtalène-2,6-diyle,
les groupes (a) et (b) pouvant être substitués par CN ou par le fluor, Z¹ et Z² représentent chacun, indépendamment l'un de l'autre -CO-O-, -O-CO- , -CH₂O- , -OCH₂- -CH₂CH₂-, -CH=CH-, -C≡C-ou une liaison simple, l'un des symboles Z¹ et Z² pouvant également représenter -(CH₂)₄- ou -CH=CH-CH₂CH₂-,
m est égal à 0, 1 ou 2,
Y représente F ou Cl et
Q représente une liaison simple, -CF₂-, -OCF₂ ou -OCHF-.

2. Utilisation des composés de formule I en tant que composants de milieux à cristaux liquides.

3. Milieu à cristaux liquides à au moins des composants à cristaux liquides, caractérisé en ce qu'il contient au moins un composé de formule I.

4. Milieu à cristaux liquides contenant, en plus d'un ou plusieurs dérivés du fluorobenzène répondant à la formule I'' 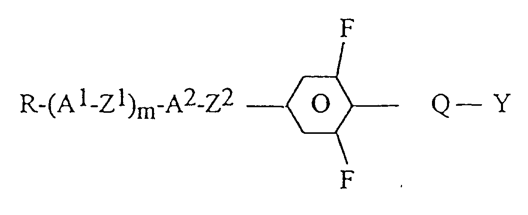 dans laquelle
R représente H, un radical alkyle ou alcènyle en C1-C15 non substitué, mono-substitué par CN ou CF₃ ou au moins mono-substitué par des halogènes, et dans lequel également un ou plusieurs groupes CH₂ peuvent être remplacés chacun, indépendamment les uns des autres, par -O-, -S-, 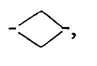 -CO-, -CO-O-, -O-CO-O- ou -O-CO-O-sous réserve toutefois que des atomes d'oxygène ne peuvent pas être reliés directement entre eux,
A¹ et A² représentent chacun, indépendamment l'un de l'autre,
(a) un groupe trans-1,4-cyclohexylène dans lequel également un ou plusieurs groupes CH₂ non voisins peuvent être remplacés par -O- et/ou -S-,
(b) un groupe 1,4-phénylène dans lequel également un ou deux groupes CH peuvent être remplacés par N,
(c) un groupe choisi parmi les groupes 1,4-cyclohexenylène, 1,4-bicyclo(2,2,2)octylène, pipéridine-1,4-diyle, naphtalène-2,6-diyle, décahydronaphtalène-2,6-diyle et 1,2,3,4-tétrahydronaphtalène-2,6-diyle, les groupes (a) et (b) pouvant être substitués par CN ou le fluor,
Z¹ et Z² représentent chacun, indépendamment l'un de l'autre -CO-O-, -O-CO--CH₂O-, -OCH₂-, -CH₂CH₂-, -CH=CH.-, -C≡C-ou une liaison simple, l'un des symboles Z¹ et Z² pouvant également représenter -(CH₂)₄- ou -CH=CH-CH₂CH₂-,
m est égal à 0,1 ou 2,
Y représente F ou Cl et
Q représente une liaison simple, -CF₂-, -OCF₂- ou -OCHF-,
de 4 à 30 autres composants.

5. Milieu selon revendication 4, caractérisé en ce que, en plus d'un ou plusieurs dérivés du fluorobenzène de formule I'' selon la revendication 4, il contient de 7 à 25 autres composants.

6. Milieu selon une des revendications 4 ou 5, caractérisé en ce que ces autres composants sont choisis parmi les composés répondant aux formules 1, 2, 3, 4 et 5
R'-L-E-R'' 1
R'-L-COO-E-R'' 2
R'-L-OOC-E-R'' 3
R'-L-CH₂CH₂-E-R'' 4
R'-L-C ≡ C-E-R'' 5
dans lesquelles
L et E, qui peuvent avoir des significations identiques ou différentes, représentent chacun, indépendamment l'un de l'autre, un groupe bivalent choisi parmi -Phe-, -Cyc-, -Phe-Phe-, Phe-Cyc-, -Cyc-Cyc-, -Pyr-, -Dio-, -G-Phe- et -G-Cyc- et leurs images spéculaires, Phe représentant un groupe 1,4-phénylène non substitué ou substitué par le fluor, Cyc un groupe trans1,4-cyclohexylène ou 1,4-cyclohexénylène, Pyr un groupe pyrimidine-2,5-diyle ou pyridine-2,5-diyle, Dio un groupe 1,3-dioxanne-2,5-diyle et G un groupe 2-(trans-1,4-cyclohexyl)-éthyle, pyrimidine-2,5-diyle, pyridine-2,5-diyle ou 1,3-dioxanne-2,5-diyle et
R' et R'' représentent chacun, indépendamment l'un de l'autre, un groupe alkyle, alcènyle, alcoxy, alcoxyalkyle, alcènyloxy ou alcanoyloxy contenant chacun 1 à 8 atomes de carbone,
R'' pouvant également représenter -F, -Cl, -NCS,
-(O)ᵢ-CH₃₋₍ₖ₊₁₎FₖCl₁ -dans lequel i est égal à 0 ou 1 et (k + 1) est égal à 1,2 ou 3 - ou -CN.

7. Milieu selon revendication 6, caractérisé en ce qu'il contient en tant qu'autres composants au moins deux composés choisis parmi ceux qui répondent aux formules 1 à 5.

8. Milieu selon revendication 6, caractérisé en ce qu'il contient un composant pour lequel L représente Cyc ou Phe et E représente Phe-Cyc.

9. Milieu selon revendication 8, caractérisé en ce que L représente Phe.

10. Milieu selon revendication 6, caractérisé en ce qu'il contient un ou plusieurs composants choisis parmi les composés répondant aux formules 1, 2, 3, 4 et 5 dans lesquelles L et E sont choisis parmi Cyc, Phe et Pyr et, simultanément, un ou plusieurs composants choisis parmi les composés répondant aux formules 1, 2, 3, 4 et 5 dans lesquelles l'un des symboles L et E représente un groupe choisi parmi Cyc, Phe et Pyr et l'autre un composant choisi parmi -Phe-Phe-, -Phe-Cyc-, Gyc-Cyc-, -G-Phe- et -G-Cyc, et le cas échéant un ou plusieurs composants choisis parmi les composés répondant aux formules 1, 2, 3, 4 et 5 dans lesquelles les symboles L et E représentent des groupes choisis parmi -Phe-Cyc-, -Cyc-Cyc-, -G-Phe- et-G-Cyc-.

11. Milieu selon une des revendications 4 à 10, caractérisé en ce qu'il contient plusieurs composés de formule I'' selon revendication 4.

12. Milieu selon revendication 11, caractérisé en ce qu'il contient trois, quatre ou cinq composés de formule I'' selon revendication 4.

13. Milieu selon revendication 6, caractérisé en ce qu'il contient (a) 5 à 90 % d'un ou plusieurs composés de formule I'' selon revendication 4, (b) 10 à 80 % de composés choisis parmi ceux qui répondent aux formules 1 à 5 dans lesquelles R'' représente -F, -Cl, -NCS ou -(O)i-CH₃₋₍ₖ₊₁₎FₖCl₁ (groupe B) et (c) 0 à 30 % de composés choisis parmi ceux qui répondent aux formules 1 à 5 dans lesquelles R' et R'' représentent chacun, indépendamment l'un de l'autre, un radical alkyle, alcènyle, alcoxy, alcoxyalkyle, alcényloxy ou alcanoyloxy en C1-C8 (groupe A).

14. Milieu selon revendication 13, caractérisé en ce qu'il contient des composés choisis parmi ceux qui répondent aux formules 1 à 5 dans lesquelles l'un des symboles L et E représente un groupe 1,4-phénylène substitué par le fluor.

15. Milieu selon revendication 13, caractérisé en ce qu'il consiste en composés de formule I'' selon revendication 4 et composés du groupe B.

16. Milieu selon une des revendications 4 à 15, sous réserve qu'un tel milieu, consistant en composés de formule I'' et composés de formule 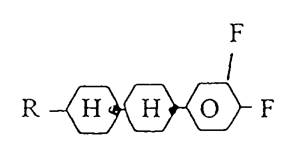 dans laquelle R représente un groupe alkyle à chaîne droite, est exclu.

17. Milieux selon une des revendications 4 à 16, caractérisés en ce qu'ils contiennent plus de 40 % de composés de formule I'' selon revendication 4.

18. Milieu selon revendication 17, caractérisé en ce que la proportion des composés de formule I'' selon revendication 4 est de 45 à 90 %.

19. Elément d'affichage à cristaux liquides caractérisé en ce qu'il contient un milieu à cristaux liquides selon une des revendications 3 à 18.

20. Elément d'affichage électro-optique caractérisé en ce qu'il contient en tant que diélectrique un milieu à cristaux liquides selon une des revendications 3 à 18.
